(19) Europäisches Patentamt
European Patent Office
Office européen des brevets

(11) **EP 3 165 156 B1**

(12) **EUROPEAN PATENT SPECIFICATION**

(45) Date of publication and mention
of the grant of the patent:
**04.03.2020 Bulletin 2020/10**

(21) Application number: **16203531.5**

(22) Date of filing: **17.11.2011**

(51) Int Cl.:
*A61B 5/0215* (2006.01)     *A61B 5/027* (2006.01)
*A61B 5/0402* (2006.01)     *A61B 5/042* (2006.01)
*A61B 5/06* (2006.01)

(54) **MEDICAL DEVICE LOCATING SYSTEM**

SYSTEM ZUR ORTUNG MEDIZINISCHER VORRICHTUNGEN

SYSTÈME DE LOCALISATION DE DISPOSITIF MÉDICAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.11.2010 US 949663
18.11.2010 US 949671**

(43) Date of publication of application:
**10.05.2017 Bulletin 2017/19**

(62) Document number(s) of the earlier application(s) in
accordance with Art. 76 EPC:
**11840907.7 / 2 640 260**

(73) Proprietor: **Teleflex Medical Devices S.à.r.l.
2220 Luxembourg (LU)**

(72) Inventors:
• **Zellers, Robert D.
Boulder, CO 80301 (US)**
• **Nelson, Peter E.
Boulder, CO 80301 (US)**
• **Henry, Charles W.
Boulder, CO 80301 (US)**

(74) Representative: **Schulz, Oliver Frank Michael
df-mp Dörries Frank-Molnia & Pohlman
Patentanwälte Rechtsanwälte PartG mbB
Theatinerstrasse 16
80333 München (DE)**

(56) References cited:
EP-A1- 1 887 309      EP-A1- 2 168 478
WO-A2-02/098273      WO-A2-2005/031282
US-A1- 2008 146 941      US-B2- 7 697 972

## Description

## BACKGROUND

In medical care, the correct placement of a medical device such as a catheter or a guide wire in a patient has become increasingly important for a number of reasons. In the case of an infusion catheter, for example, medications can need to be targeted to, or for, specific organs, or areas of the body. A catheter can need to be located sufficiently near the heart in a particular region where there is a particular blood flow rate; as for example, a particular high blood flow rate to ensure adequate dilution/mixing of infused fluids. Alternatively, a catheter or other internally-positioned medical device can simply need to be disposed in the right place to function; as for example, an enteral feeding tube within the stomach. Use of a medical device position location and/or guidance system can thus provide for less skilled practitioners to accurately and reliably position a medical device such as a catheter.

Accordingly, a variety of systems have been developed to attempt to indicate location or position of catheters within the body of a patient. Relatively reliable location devices have made use of x-ray or fluoroscopy; however, these devices expose the patient and/or caregiver to undesirable amounts of radiation. As a consequence, a variety of different systems have been attempted to more continuously and accurately indicate location of a catheter with a goal of replacing the use of x-rays. However, such systems still suffer from various problems.

Electromagnetic catheter position location devices have been in development. Some position location systems have made use of alternating current, AC, driven external coils with a sensor (sensor coil) in the catheter tip. Others have used an AC driven coil in the catheter tip with external sensor coils. A disadvantage of such a conventional catheter tip driven system has been the need for heavy or thick wires running into the catheter to carry sufficient drive current to generate a sufficient electromagnetic signal for the external sensors. This has precluded the use of such a system with smaller diameter catheters. Other position location systems have used a fixed (or DC) magnet on the catheter tip with external sensor coils. A significant disadvantage to such a fixed magnet location system has been that the magnet would necessarily be very small, and as such would generate a very small signal from the tip of the catheter. As a consequence, other magnetic fields in the vicinity can create significant interference problems for such a system. Furthermore, the field of such a magnet drops off extremely quickly over distance and thus cannot be sensed more than a few inches deep into the patient's tissue. Another AC drive system has been described including driving two coils simultaneously; however, those respective coils were specified as having been driven at two different frequencies so that the coil drives arc not additive and the sensor demodulates the two different frequencies as two independent values. Yet another AC drive system has been described driving two coils simultaneously in quadrature which simulates a single spinning coil; however, this system can only indicate the orientation of the sensor in the x-y plane and its relative position in that plane.
EP 1 887 309 A1 and WO 02/098273 A2 disclose systems locating a medical device inside a patient using multiple coil triplet, wherein the coils are driven with different frequencies in order to be distinguishable. EP 2 168 478 A1 discloses a method directed to detection and compensation for artifacts experienced during position sensing of a probe in a living body. US 7,697,972 B2 relates to systems and methods for using medical images to assist in navigating an instrument through internal body structures,

This statement of background is for information purposes only and is not intended to be a complete or exhaustive explication of all potentially relevant background art.

## SUMMARY

An improved system for locating the disposition of a sensor coil in a subject according to the invention is defined by claim 1.

Devices, methods and systems of the present developments can include a sensor coil that may be associated with a medical device such as a catheter, this sensor coil being communicatively cooperative with, or responsive to an array of drive coil sets of drive coils placed relative to a subject's body to allow detection or positioning of the medical device in the subject's body. Each of the drive coil sets and the sensor coil may also be communicatively connected or cooperative with an external control and/or display box, for selective driving of the drive coils of the sets of drive coils and for receiving response signals from the sensor coil.

Methods, devices and systems can be provided for one or both of two- or three-dimensional location of the disposition of a sensor coil in a subject including: an array of electromagnetic drive coil sets, each set having two or three dimensionally oriented drive coils; a sensor coil being electromagnetically communicative with the array of electromagnetic drive coil sets; and, a system controller communicative with and adapted to energize one or more of the electromagnetic coils in the array of electromagnetic drive coil sets, the energizing of the one or more of the electromagnetic coils including one or more of energizing the coils singly, or in pairs of x-y and y-z or x-z coils while measuring the response of the sensor coil; whereby the system uses the measurements of the responses of the sensor coil to calculate the location and orientation of the sensor coil relative to said drive coil sets.

These and still further aspects and advantages of the present developments arc illustrative of those which can be achieved by these developments and are not intended to be exhaustive or limiting of the possible advantages which can be realized. Thus, these and other aspects and advantages of these present developments will be apparent from the description herein or can be learned from practicing the disclosure hereof, both as embodied herein or as modified in view of any variations which can be apparent to those skilled in the art. Thus, in addition to the exemplary aspects and embodiments described above, further aspects and embodiments will become apparent by reference to and by study of the following descriptions, including as will be readily discerned from the following detailed description of exemplary implementations hereof especially when read in conjunction with the accompanying drawings in which like parts bear like numerals throughout the several views. The present invention is defined solely by the appended claims.

## BRIEF DESCRIPTION OF THE DRAWINGS

In the drawings:

Fig. 1 is a schematic overview of present developments showing a user control box, and a patient drive coil block with drive coil sets, and a guide wire or stylet with a sensor coil and cable.

Fig. 2 is a detailed view of one three-axis drive coil set.

Fig. 3 is a detailed view of a sensor coil.

Fig. 4 is an illustration of magnetic vectors generated by a normal coil drive.

Fig. 5 is an illustration of orthogonal magnetic vectors generated by an x-y virtual drive.

Fig. 6 is an illustration of orthogonal magnetic vectors generated by a y-z virtual drive.

Fig. 7 is a detailed view of a drive coil and sensor coil block with an addition of an optional ECG measurement.

Fig. 8 is a block diagram of a present development hereof.

Fig. 9 is a block diagram of a display/interface and user control box.

Fig. 10 is a block diagram of a function of a main interface board in a user control box.

Fig. 11 is a block diagram of an overall function of a patient drive coil block.

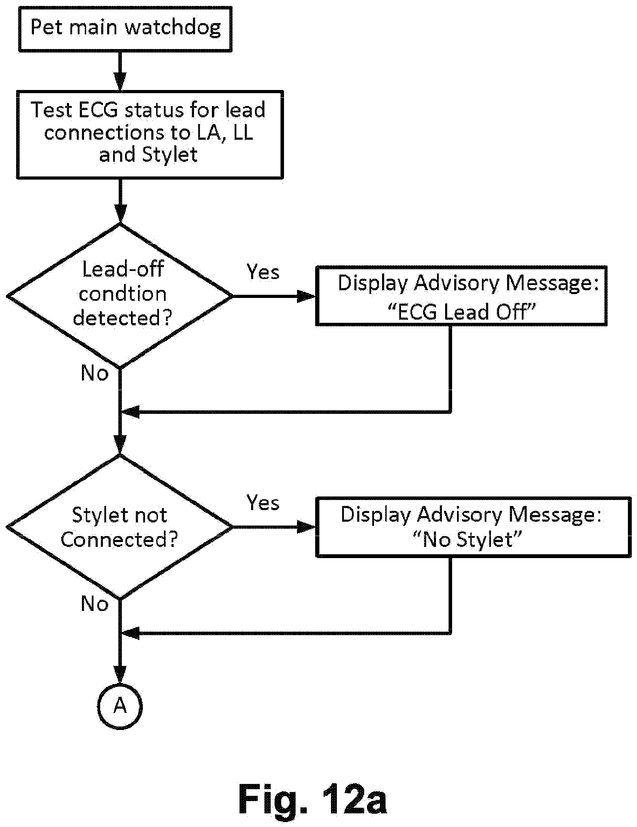

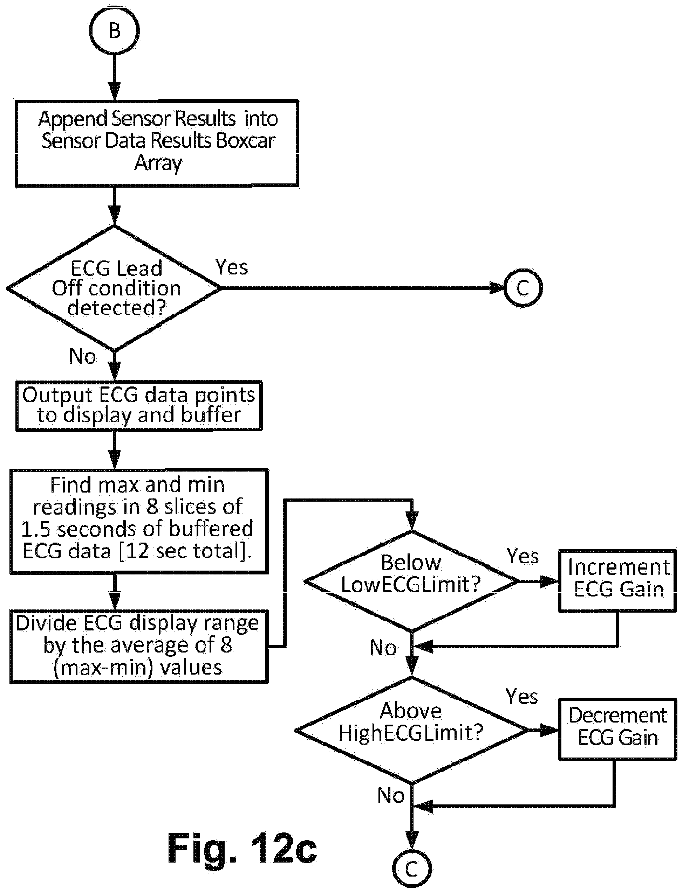

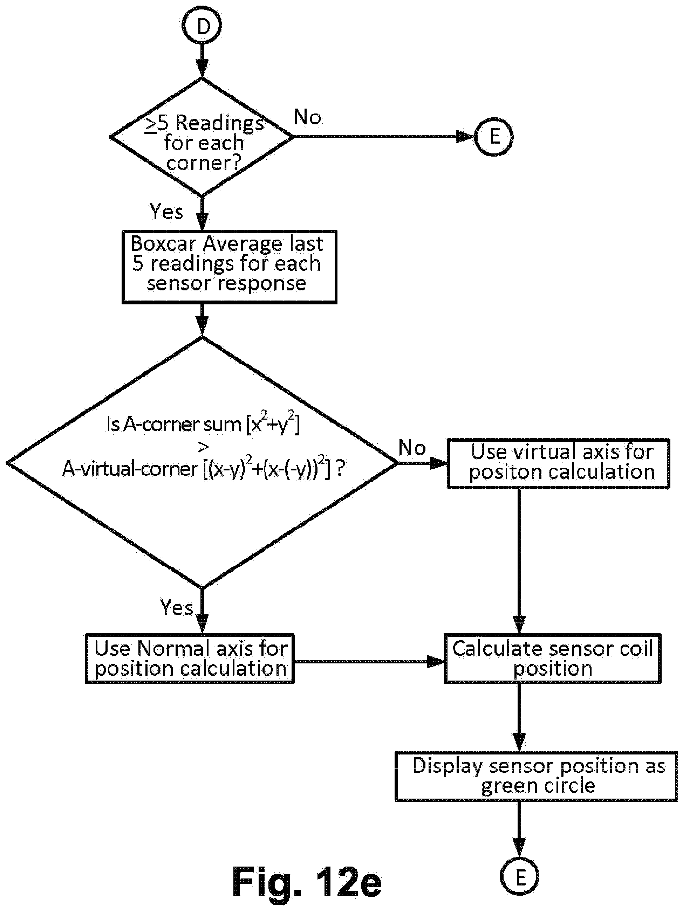

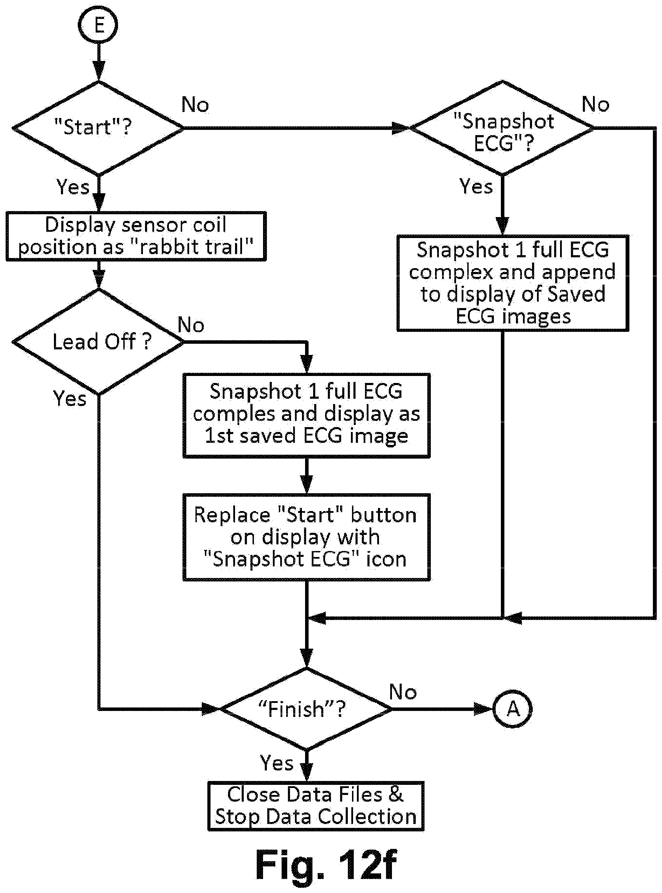

/fig. 12a-12f detail algorithms for controlling an acquisition of sensor coil position optionally including display and/or ECG.

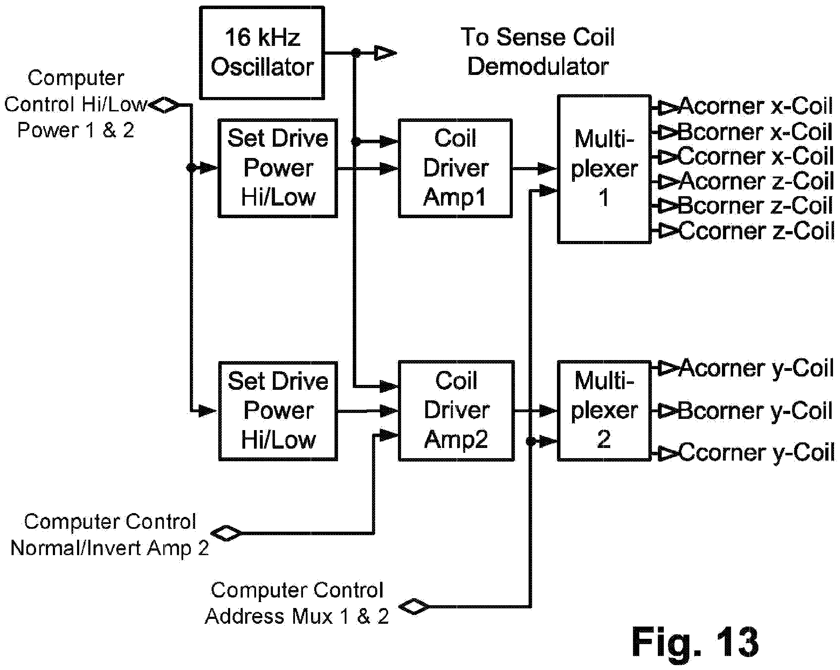

Fig. 13 is a detailet block diagram of function of a coil drive with virtual x-y capability.

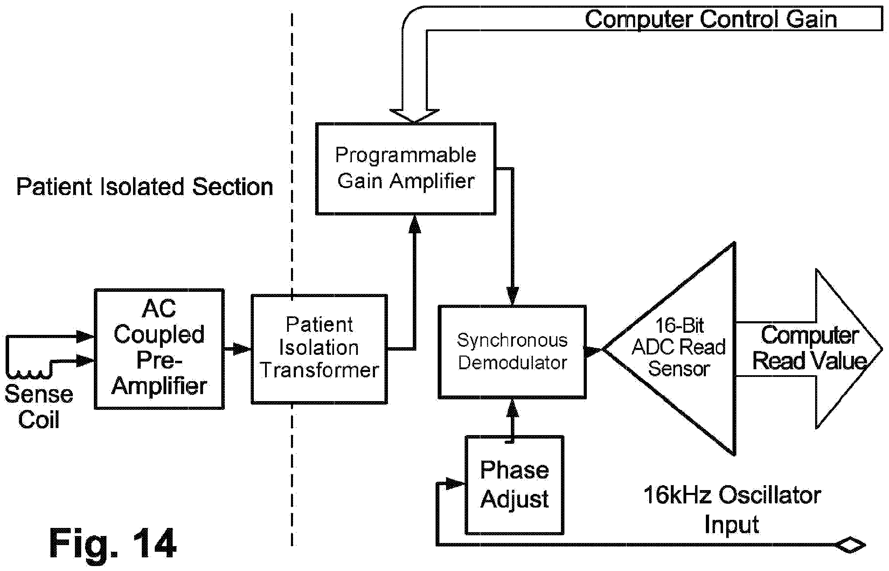

Fig. 14 is a detailed block diagram of sensor coil signal processing.

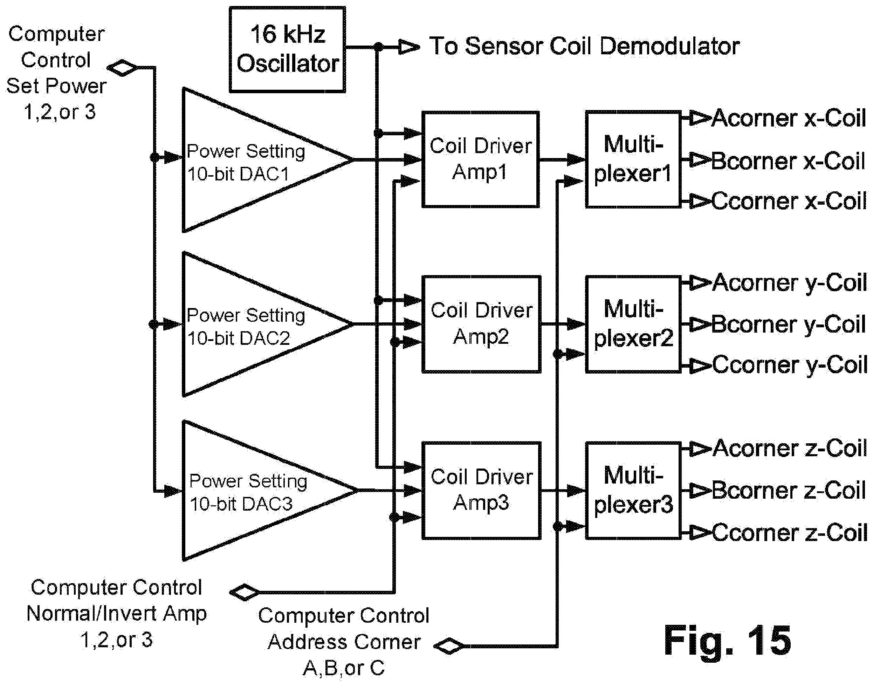

Fig. 15 is a detailed block diagram of a function of a coil drive with full virtual x-y-z capability.

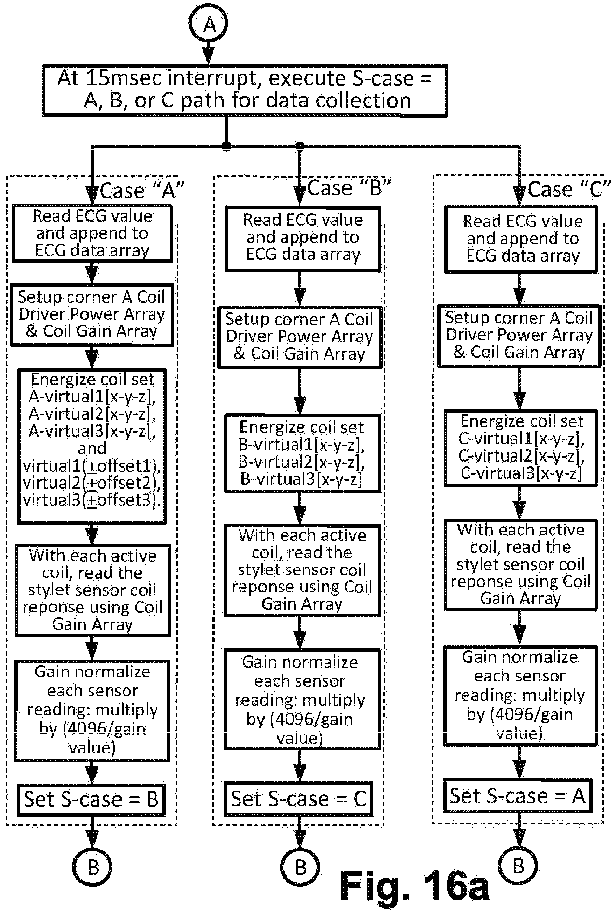

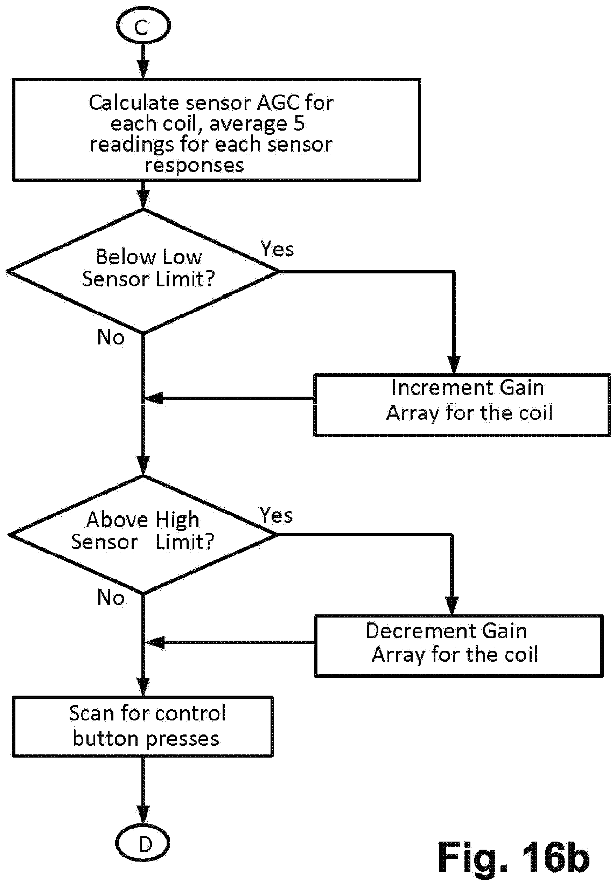

Fig. 16a-16c detail some algorithm alternatives for controlling the acquisition and display of sensor coil position in virtual x-y-z system.

## DETAILED DESCRIPTION OF FIGURES

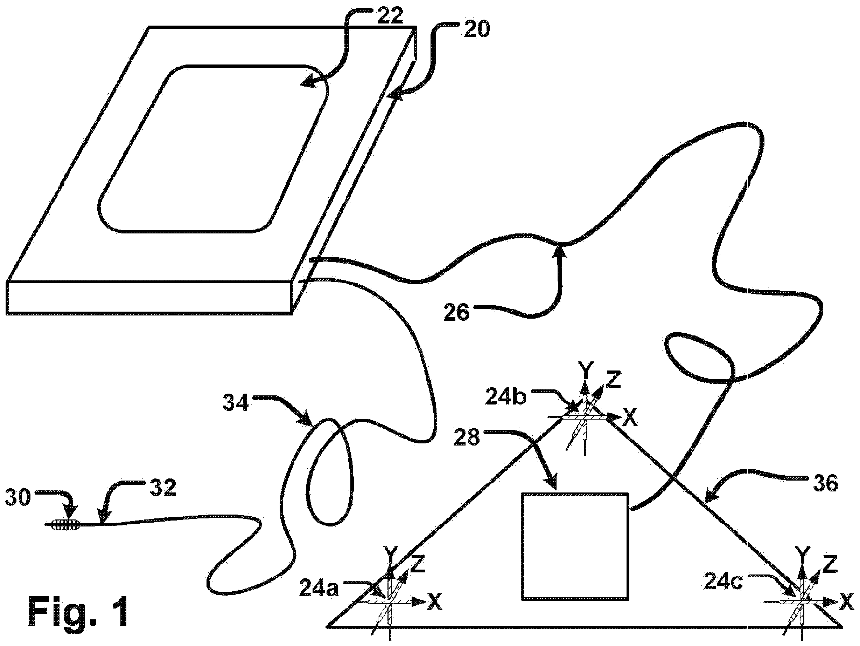

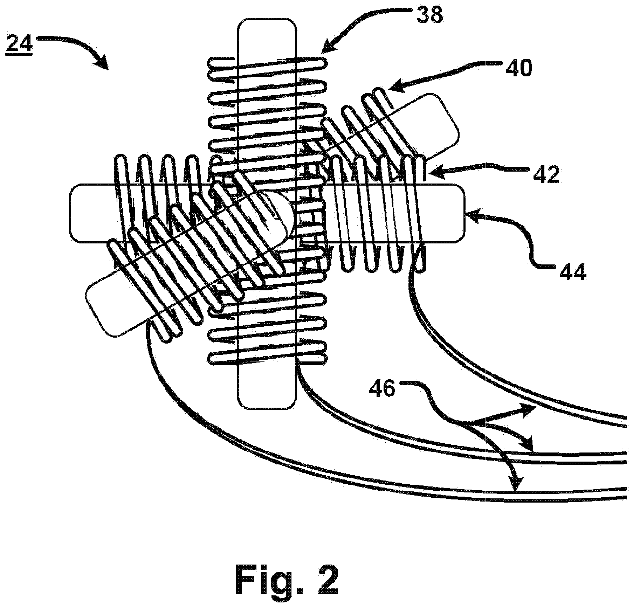

Figure 1 provides an overview of an implementation of a medical device location system hereof. A user control box **20** may be included, and according to this implementation, contains a touch screen display **22,** a single-board computer (SBC) (not separately shown in Fig. 1) for control and data processing, and a main interface board (also not separately shown in Fig. 1) which connects to a drive block cable **26** and a medical device cable **32** (also sometimes referred to as a catheter, or guide wire or stylet cable **32**). A triangular patient drive coil block **36** may be connected via drive block cable **26** to the control box **20** (the drive coil block also sometimes being referred to as an emitter block, a patient block or merely a drive block). Coil drive electronics **28** and three drive coil sets **24a, 24b, 24c** (also sometimes referred to as emitter coils, or x-y-z drive coils) are mounted in the drive block **36.** The coil drive electronics **28** allow the SBC to selectively energize any drive coil axis **38, 40, 42** of a set **24** (see Fig. 2) or group of drive coil axes. A sensor coil **30** is, in this implementation, built on or within the tip of a medical device such as a small diameter biocompatible guide wire or stylet cable **32.** The guide wire may then be placed in the patient and the catheter then threaded over this wire; or, alternatively, the stylet cable may then be inserted up to the distal end of a catheter before the catheter is placed in the patient. A two-conductor cable **34** can be used to connect the sensor coil of guide wire or stylet back to the user control box.

Figure 2 shows a detailed drawing of a drive coil set **24** (representative of any of sets **24a, 24b, 24c).** Here, the x-coil **42,** the y-coil **38** and z-coil **40** each have a ferrite or ferrous core **44** to enhance the magnetic field generation. This figure is only schematically representative of the construction of a drive coil; in actuality, each drive coil may have many windings (e.g. 100 turns) on the ferrite core and can be constructed as three (3) coil pairs to facilitate the intersection of the x, y, and z axes. Each coil here has a set of lead wires **46** to connect back to the multiplexers of the coil drive electronics **28.**

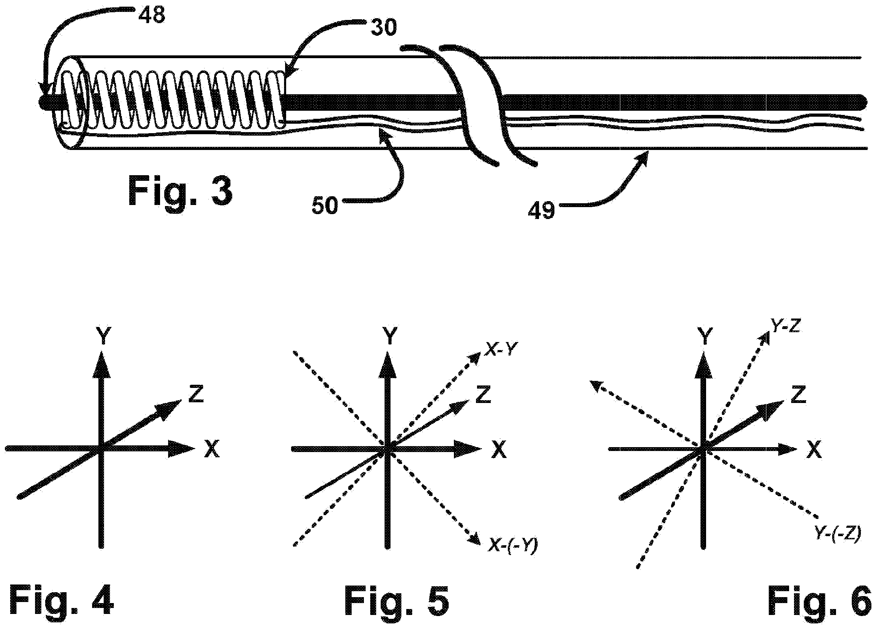

Figure 3 shows a detailed view of an exemplar medical device; e.g., a guide wire or stylet sensor coil. The sensor coil **30** can be any suitable gauge (e.g., but not limited to, a very fine gauge (e.g. 0.001" diameter)) insulated wire wound around a ferrous core wire **48.** This figure is schematically illustrative only of the sensor coil; here, the sensor coil is approximately 400 turns in single layer, but can be any suitable turns per length, e.g., 50-1000, or any range or value therein, e.g., 100, 200, 300, 350, 400, 450, 500, 550, 600, 650, 700, and the like. An alternative construction can optionally be 400 or more turns in 2, 3, 4, 5, or more layers; here, the advantage of multiple even layers may be the lead wires come off the same end of the coil. The sensor coil lead wires **50** connect back through a cable to the patient isolated portion of the main interface board. A thin insulating tubing **49** can be used to provide a protective sleeve for the whole assembly.

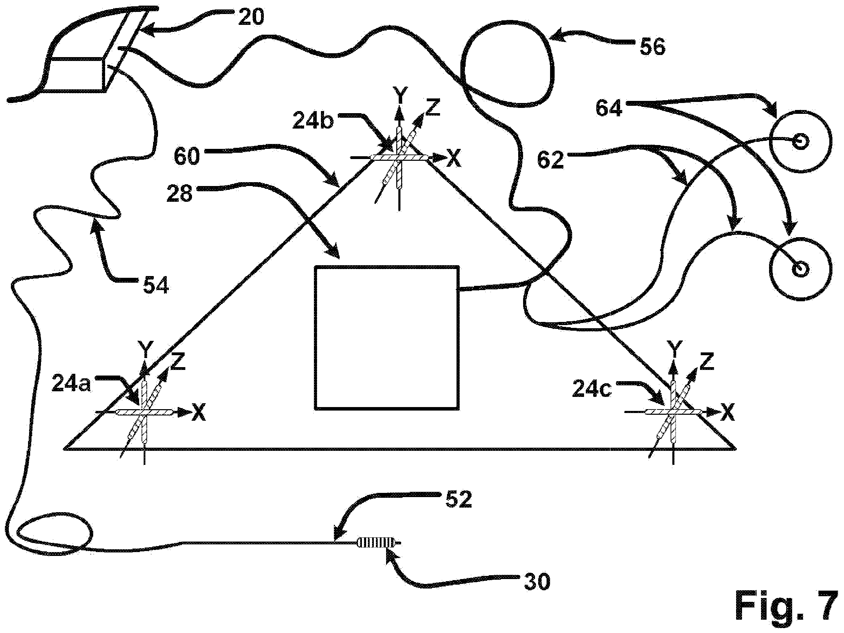

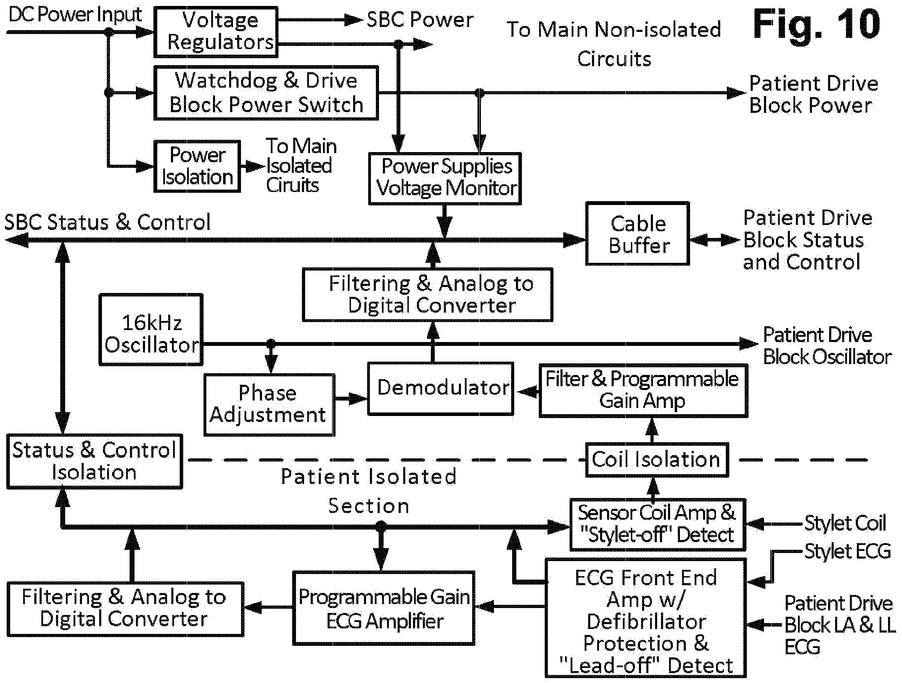

In an optional ECG version of a catheter location system (e.g., Figure 7), the tip of the ferrous, conductive core wire **48** can be polished smooth and can provide an electrical signal as an ECG lead from within the catheter. In such aversion, the guide wire or stylet sensor connection is accomplished with three wires, two (2) for the coil sensor and one (1) for the ECG, through a cable **54** to the patient isolated portion of the main interface board in the user control box **20**. Furthermore, the patient drive coil block **60** can have two ECG pads added which connect through a cable **62** to the patient isolated portion of the main interface board. These two ECG inputs together with the one ECG from the catheter provide a three-lead ECG measurement system (e.g., see Figure 10).

Figures 4, 5 and 6 illustrate an optional version of the operation of a normal-drive and virtual-drive drive coil set. Figure 4 shows magnetic vectors x, y, and z generated by normal coil driving of the x-axis coil **42,** the y-axis coil **38** and z-axis coil **40** (as shown in Figure 2). Figure 5 shows the virtual magnetic vector x-y generated by simultaneously driving the x-axis coil **42** and the y-axis coil **38** (as shown in Figure 2) and when both are driven at the same power, the vector is forty-five degrees between the x and axes. The virtual magnetic vector x-(-y) generated by simultaneously driving the x-axis coil **42** and the phase-inverted, y-axis coil **38** (as shown in Figure 2) and when both arc driven at the same power, the vector is minus forty-five degrees between the x and -y axes. If a digital to analog converter (DAC) is added to control power to the x-axis drive and another DAC added to control power of y-axis drive, then it is possible to point the virtual axis to any angle from 0 to 360 degrees between x and y. For example, if x-axis power DAC is maximum and y-axis power DAC is 1/4$^{th}$ (one quarter) of maximum then the vector sum of x-y drive yields a virtual axis of approximately fourteen degrees between the x and y axes. Figure 6 shows a virtual magnetic vector y-z generated by simultaneously driving the z-axis coil **42** and the y-axis coil **38** (as shown in Figure 2); and a virtual magnetic vector z-(-y) generated by simultaneously driving the z-axis coil **42** and a phase-inverted, y-axis coil **38** (as shown in Figure 2). These figures illustrate the simplest and one optional form of virtual drive (e.g., see Figure 13), it is possible to point a virtual magnet vector to any polar coordinate by simultaneously driving x, y, and z coils at independent power levels (e.g., see Figure 15).

Figure 7 provides a schematic diagram of an optional medical device location system with optional electrocardiograph (ECG) measurement. This is similar to the Figure 1 implementation with the addition of three ECG leads. The user control box **20** connects through a main interface board to the drive block cable **56** and guide wire or stylet cable **54**. The patient drive block **60** may be connected via drive block cable **56** which includes two isolated ECG lead signals to the control box **20**. The coil drive electronics **28** and three x-y-z drive coils **24a, 24b,**

**24c** may be mounted in the drive block **60**. The coil drive electronics **28** allow the single board controller (SBC) to selectively energize any drive coil axis **38, 40, 42** (Fig. 2) or group of drive coil axes. Two ECG pads **64** are placed on the patient and connected by ECG lead wires **62** to the drive block **60**. The guide wire or stylet sensor **30** here is built onto a small diameter biocompatible conductive-tip guide wire or stylet cable **52** which is inserted into a catheter before (stylet) or after (guide wire) the catheter is placed in the patient. A three-conductor cable **54** connects the guide wire or stylet sensor coil and one ECG lead back to the user control box **20**.

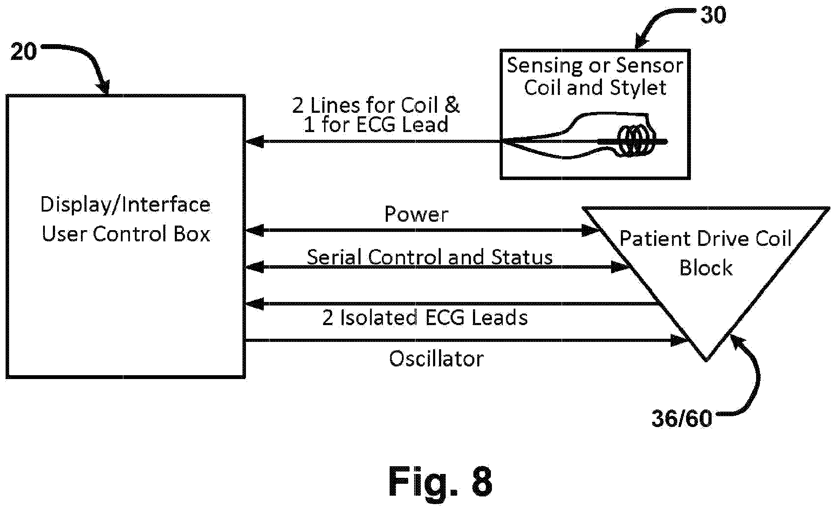

Figure 8 is an overall schematic diagram of a medical device location system. This figure illustrates connections between a user control box **20** and a sensing guide wire or stylet **30** and a patient drive block **36, 60.** The control box **20** can include an integrated, separated, or remote user display and/or interface. Each of these components can include cables or connectors for one or more of a coil interface, a power supply, a serial interface, a control interface, a status interface, an ECG interface or lead, an oscillator interface, a processor interface, a computer interface, a data interface, a network interface (cable or wireless), an internet interface, a video interface, a touch-screen interface, an SBC control or power interface, a board interface, a sensor interface, an isolator interface, and/or the like as described herein or as known in the art. One or more of these cables or connectors can attach to the patient drive block **36/60** or any component thereof.

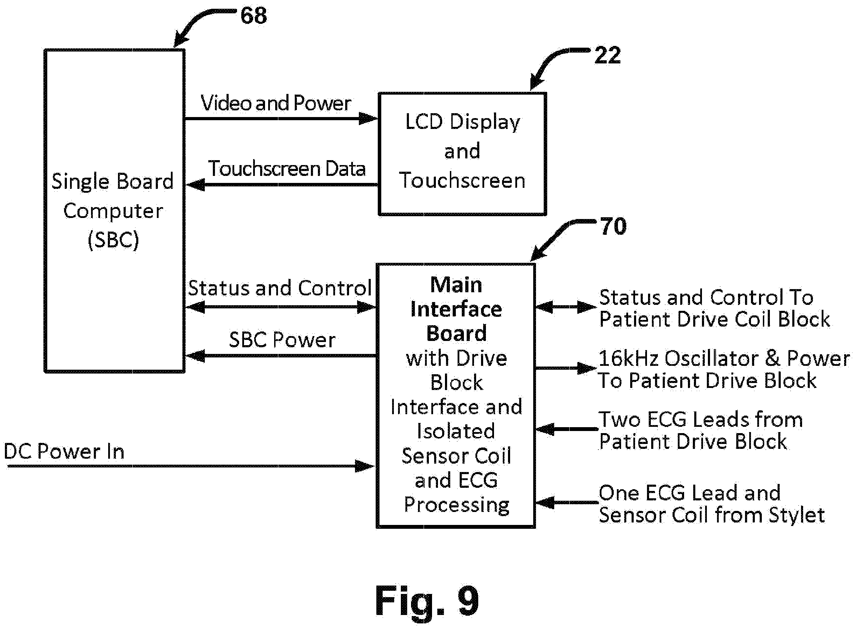

Figure 9 is a block diagram of a user control box **20** which in this implementation includes a computer **68,** an LCD display **22** with touch-screen, and a main interface board **70.** Each of these components can include cables or connectors for one or more of a coil interface, a power supply, a serial interface, a control interface, a status interface, an ECG interface or lead, an oscillator interface, a processor interface, a computer interface, a data interface, a network interface (cable or wireless), an internet interface, a video interface, a touch-screen interface, an computer control or power interface, a board interface, a sensor interface, an isolator interface, and/or the like as described herein or as known in the art.

Figure 10 provides a detailed block diagram of a main interface board **70** (Fig. 9) and shows a patient isolated section which connects to a guide wire or stylet cable **34, 54** and an ECG leads from a patient drive block **60.** The remainder of the circuitry controls power/interface to a patient drive block **36, 60** and power to a single board computer **68,** including one or more of a voltage regulator, a watchdog swith, a drive switch, a power isolator, a voltage monitor, a cable buffer, a filter, an analog to digital converter, a phase adjuster, a demodulator, a signal filter, a programmable gain amplifier, a coil isolator, a coil sensor coil amplifier, a detector, memory, flash memory, a multiplexor, a polarity inversion switch, and/or the like. Each of these components can include cables or connectors for one or more of a coil interface, a power supply, a serial interface, a control interface, a status interface, an ECG interface or lead, an oscillator interface, a processor interface, a computer interface, a data interface, a network interface (cable or wireless), an internet interface, a video interface, a touch-screen interface, an SBC control or power interface, a board interface, a sensor interface, and/or the like as described herein or as known in the art. One or more of these cables or connectors can attach to a patient drive block **36/60** or any component thereof.

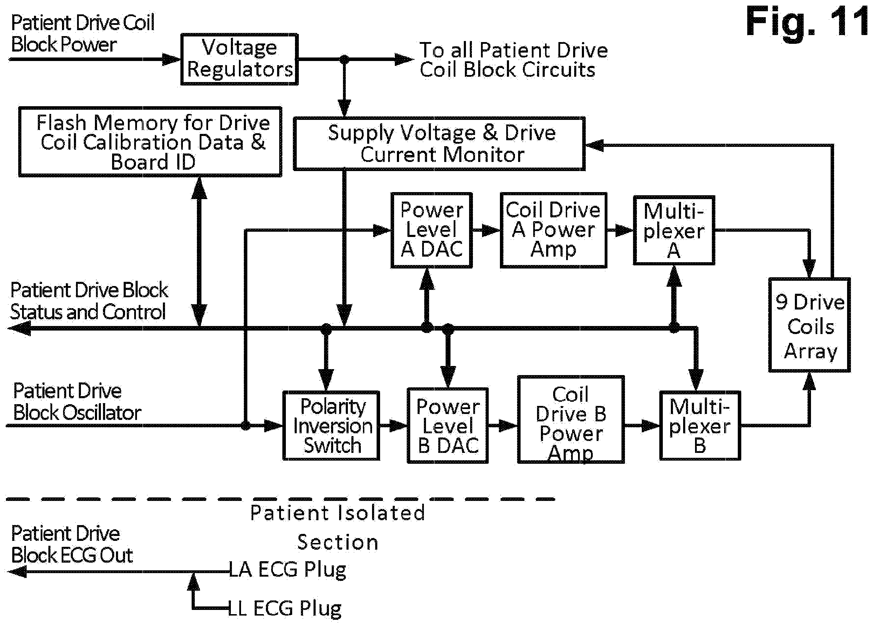

Figure 11 is a detailed block diagram of a patient drive block **36, 60** (Figs. 1, 7). A simpler version/option of a patient drive block **36** does not have ECG therefore no ECG leads; whereas, patient drive block **60** may include two ECG leads with patient isolation. The drive coil drive system in this diagram illustrates a two-coil virtual drive capability allowing the computer software to simultaneously drive two coils at select power levels. Such a system can include one or more of a voltage regulator, a watchdog switch, a drive switch, a power isolator, a voltage monitor, a cable buffer, a filter, an analog to digital converter, a phase adjuster, a demodulator, a signal filter, a programmable gain amplifier, a coil isolator, a coil sensor coil amplifier, a detector, memory, flash memory, a multiplexor, a polarity inversion switch, and/or the like. Each of these components can include cables or connectors for one or more of a coil interface, a power supply, a serial interface, a control interface, a status interface, an ECG interface or lead, an oscillator interface, a processor interface, a computer interface, a data interface, a network interface (cable or wireless), an internet interface, a video interface, a touchscreen interface, an SBC control or power interface, a board interface, a sensor interface, and/or the like as described herein or as known in the art.

Figures 12a-f provide an overview of a software functionality for a medical device location system hereof.

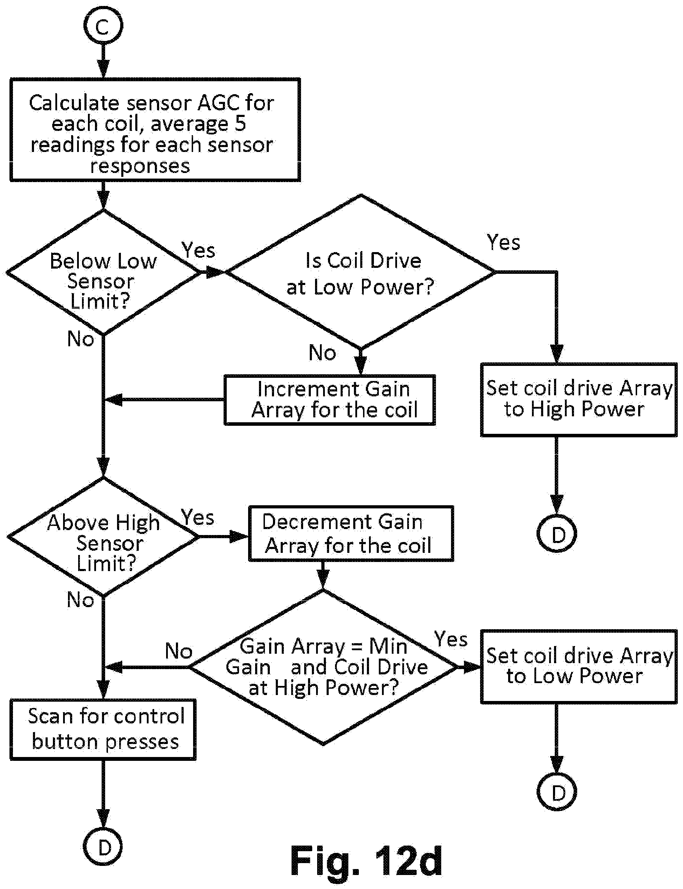

Figure 13 is a simplified virtual coil drive system located on a patient drive block **36, 60.** A coil driver hereof may have only two coil drives and only high/low power selection instead of a power control DAC. In this drive system it is possible to generate x-y and x-(-y) virtual coil drive (see Figure 5) and also z-y and z-(-y) drive (see Figure 6). One additional feature of this drive is a low power setting which allows drive power reduction if the sensing coil is too close to the drive coil (see software flow chart Figure 12d). Tt also is possible to have two additional virtual vectors in this drive system by driving x-high-power together with y-low-power or driving x-low-power with y-high-power. Here, driving x-low-power together with ylow-power yields the same virtual axis - forty-five degrees from x and y axes - as driving both at high power.

Figure 14 is a detailed view of the sensor coil processing system of the main interface board **68.** The sensor coil **30** on the guide wire or stylet can be connected through a cable **34, 54** to the main interface board **68.** This sensor coil input is pre-amplified and filtered then passed through a patient isolation transformer to a software-controlled programmable gain amplifier. This amplified signal is then demodulated using the low frequency (e.g. 16kHz) drive oscillator. The software then reads the sensor coil value with a high resolution (e.g. 16 bit or higher resolution) analog to digital converter (ADC). This read value for each drive coil activated and this value is proportional to the magnetic field measured by the sensor coil during that drive coil activation.

Figure 15 is a detailed view of a more complex virtual drive system. This drive system allows the x-axis coil **42,** the y-axis coil **38** and z-axis coil **40** to all be driven simultaneously at independent power levels set by computer control through individual digital to analog converters (DAC). In this drive system, the virtual magnetic vector is the vector sum of x-axis drive plus y-axis drive plus z-axis drive. This virtual drive permits the virtual vector to point to any polar coordinate in space, and thus use polar coordinates as an option; however, it may often still be preferable to use a set of three orthogonal "virtual" axes to calculate the sensor coil **30** position.

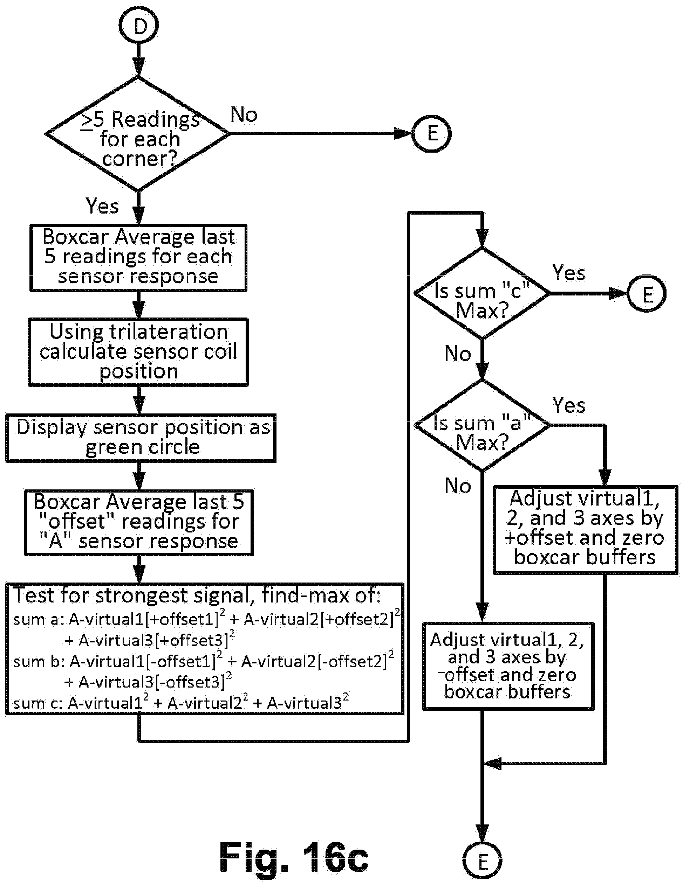

Figures 16a-c are a software flow chart showing changes drive and sensor coil processing for a fully independent x-y-z virtual drive system (see Figure 15). This software adds a positive offset test and a negative offset test to the virtual axes for the A-corner coils. If the sensor coil response is stronger for an offset axis (Figure 16c) than the current virtual axes, the system shifts to use the offset axes.

**DETAILED DESCRIPTION**

An aspect of the present developments is to provide an accurate system to generate a three-dimensional indication of location, position, orientation and/or travel of a medical device such as a guide wire or a catheter or stylet placed within a patient. This can in some implementations include a display of the location and/or travel of the device. A system hereof can include a sensor coil which is disposed in or on the tip of a guide wire or stylet cable, this sensor coil being communicatively cooperative with an external control and/or display box which may also be communicatively connected with an array of three-axis drive coils placed in some implementations in a triangular block on the patient's chest. This is also sometimes referred to as a drive block or an emitter block. The block contains the coil-drive controller that facilitates driving single coils, or pairs of coils, or triplets of coils together. The pair driving allows x-y, x-z, or y-z coils in a corner to be energized at the same frequency and same power creating a virtual drive axis at a 45 degree angle between the axis pairs. The coil-drive may also have an additional control to invert the drive waveform (shift the phase 180 degrees). This inversion of one coil in the pair can create a virtual drive axis at -45 degrees, thus creating an orthogonal pair of virtual axes within a plane. For example, the virtual x-y and x-(-y) are in the same plane as the x and y axes but rotated 45 degrees within the plane. This paired drive scheme improves the measurement accuracy of the system, especially when the sensor inside the catheter tip is substantially or exactly perpendicular to a normal coil drive axis. The system controller sequentially drives/energizes each coil, then each pair of coils while measuring the sensor coil response. When the sensor coil is nearly perpendicular to a drive axis there is significantly diminished response; thus, the virtual axis measurement will provide more accurate data for the position algorithm. Algorithms within the controller can be used to select the best data sets - regular x-y-z axis or virtual x-y-z axis - to calculate the sensor/medical device (e.g., catheter tip) location, position and/or orientation. A display can be used to show the catheter tip location as a position track of x-y location plotted over time plus an indicator for the z-axis, depth of the catheter. Depth could also be indicated by a variety of methods, as for example by thickening the position line segment in the plot as z decreases and thinning the position line segment as z increases. Alternatively, depth can be displayed as a lateral or "depth" view as a position track of y-z location plotted over time.

It is possible to extend this system further using programmable current control to the coil-driver circuit. Here, a virtual axis could be created at any angle between a magnet pair in a corner by energizing two magnets at the same frequency but with different current drive (power) levels to yield a vector-sum virtual axis at any angle between 0 and 90 degrees and inverting one coil in this drive scheme to yield a vector-sum virtual axis at any angle between 0 and -90 degrees. However, an orthogonal set of axes would typically still be selected to accurately locate the sensor coil. A further extension to this system could include energizing all three electromagnetic coils, x-y-z, together in a corner using the programmable current controls and inversion controls. The result here would be a vector sum from x-drive, y-drive, and z-drive coils that creates a virtual drive at any vector within three-dimensional space.

It is also possible to enhance this system with the integration of electrocardiogram (ECG) display with the location system. Here two reference electrodes may be plugged into or otherwise connected to the triangular patient block with the third electrode connected to the stylet or guide wire. An ECG may then be displayed for the user, so that P-wave changes, or other waveform changes can be shown to indicate proximity of the stylet or guide wire to the heart.

An aspect of the present developments is an electromagnetic medical device locating system for locating a medical device or the end or the tip thereof in a subject, including one or more of:

> three or more triplet drive coil sets, each drive coil set including at least three orthogonally arranged discrete drive coils, each of the discrete drive coils being electromagnetic (EM) coils;
> at least one sensor coil;

one or more system components that one or both provide drive signals energizing said discrete drive coils and measure resulting sensor coil response signals; wherein the provision of drive signals includes one or both:

> (i) sequentially driving one or pairs of said discrete drive coils within a triplet drive coil set; and
> (ii) selectively providing phase inversion of the drive signal to any one or pairs of said discrete drive coils within a triplet drive coil set;

a computing component for calculating sensor coil disposition in the subject relative to said triplet drive coil sets from one or more measured resulting sensor coil response signals.

Another aspect may include: (a) three or more virtual or actual x-y-z axis electromagnetic (EM) triplet drive coils, each including at least three virtual or actual EM drive coils arranged in perpendicular axis to each other along an x-y-z axis, the virtual or actual EM triplet drive coils placed in a two- or three-dimensional geometric array; (b) at least one medical device sensor coil in physical association with at least one medical device tip and connected to at least one demodulator circuit; (c) at least one AC drive controller that (i) drives sequentially one or more of the virtual or actual EM drive coils; and (ii) provides a phase shifted signal to the demodulator; (d) at least one demodulator circuit including at least one demodulator for measuring the sensor coil output signal using frequency correlation with at least one AC coil driver signal from the AC drive controller to provide a synchronously demodulated sensor coil signal; (e) at least one automatic gain control circuit that maximizes the demodulated sensor coil signal; (f) a computing component for normalizing the resultant demodulated sensor coil signal data by dividing or multiplying the determined programmable gain value from the measurement of the demodulated sensor coil signals; (g) a computing component for selecting and calculating the optimized demodulated sensor coil signal data set generated from demodulated sensor coil signals, which optimized coil signal data set is calculated based on the sum of measured squared terms that have relatively higher or highest values; (h) a calculator for calculating the distance of the sensor coil and medical device tip from three or more virtual or actual EM triplet drive coil locations using the optimized demodulated sensor coil data set calculated using intersection of the spheres to provide the location of the sensor coil and corresponding medical device tip in space relative to the location of two or more of the virtual or actual EM triple drive coils provided in the two- or three-dimensional geometric array corresponding to the location of the medical device tip in the subject; and in some implementations, (i) a display. The result is the actual location of the medical device tip in the subject indicating height, width, and depth of the medical device tip in the subject calculated relative to the position of the drive coil geometric array.

If is possible to extend this system to include wherein one or more of (i) the virtual or actual EM drive coils or the virtual or actual EM triplet drive coils are arranged outside at least one of a two dimensional plane defined by at least three of the virtual or actual EM triplet drive coils; and/or (ii) at least four of the virtual or actual EM triplet drive coils form a tetrahedron as part of the three-dimensional geometric array.

It is possible to enhance such a system to include one or more of wherein (i) the display displays the relative location of the sensor coil or the medical device tip as a tracking of the sensor coil or medical device tip location over time; (ii) the display displays the sensor tip angle graphically for the user of the system, wherein the medical device tip angle is the angle of maximum response of the sensor coil as measured from sweeping the virtual drive axis through x-y plane (e.g. 0 to 360 degrees) then using this x-y maximum response angle as a vector added to the sweep through the virtual z axis; and/or (iii) the display displays the sensor tip angle graphically for the user of the system, wherein the medical device tip angle is the angle perpendicular to the angle of minimum response of the sensor coil as measured from sweeping the virtual drive axis through x-y plane (e.g. 0 to 360 degrees) then using this x-y minimum response angle as a vector added to sweep through the virtual z axis.

It is possible to extend such a system to further include wherein the intensity or power of current running through one or more adjacent EM drive coils in at least one of the EM triplet drive coils is programmable or adjustable using a control box including a programmable computer.

An aspect of the present developments is to provide a system wherein one or more of the EM drive coils are provided as the virtual EM drive coils, and wherein: (a) one or more controllers that select pairs or triplets of drive current values of the EM drive coils at regular time intervals to provide one or more paired magnetic drive coil vector values at angles from 0 to 90 degrees and phase inversion of at least one of the corresponding EM drive coils in at least one pair of the paired or tripled magnetic drive coil vectors to further provide one or more magnetic drive coil vector values at angles from -90 to 0 degrees; (b) one or more controllers that: (i) determine the angle values of maximum and minimum sensor coil responses within a plane using paired coil programmable current drive sweeping a range from 0 to 90 degrees, and (ii) that then determine the angle values of maximum and minimum sensor coil responses within a plane using paired coil programmable current drive sweeping using inverted phases of at least one coil and sweeping a range from -90 to 0 degrees; and/or (c) a calculator that: (i) computes at least one set of optimal virtual drive x and y axes for at least two of the EM triplet drive coils as values corresponding to plus and minus 45 degrees from the maximum and minimum sensor coil responses; and (ii) computes an optimal virtual drive z axis orthogonal to the plane of the optimal virtual drive x and y axes to provide at least one optimal virtual EM triplet drive axes and at least one of the virtual EM triplet drive coils.

Such a system can be extended wherein the system includes a programmable coil drive current for each of x, y, and z drive coils driven; and wherein (a) the triplets of drive current values selected at regular time intervals are provided as (i) paired magnetic drive coil vector values at angles from 0 to 90 degrees in an x-y plane together with 0 to 90 degrees from the x-y plane to the corresponding z-axis; and (ii) as phase inversion of one or two paired magnetic drive coil vector values at angles from -90 to 0 degrees in an x-y plane together with from -90 to 0 degrees from the x-y plane to the corresponding z-axis; (b) the angle values of maximum sensor coil responses within a plane for both 0 to 90 and -90 to 0 degrees are fixed and used for at least two x and y virtual axes in a virtual plane and the values of maximum sensor coil response are determined for the corresponding virtual z axis to provide at least one maximum virtual x-y-z vector for at least one of the corresponding virtual EM triplet drive coils; (c) the angle values of minimum sensor coil responses within a plane for both 0 to 90 and -90 to 0 degrees are fixed and used for at least two x and y virtual axes in a virtual plane and the values of maximum sensor coil response arc determined for the corresponding virtual z axis to provide at least one minimum virtual x-y-z vector for at least one of the corresponding virtual EM triplet drive coils; (d) the at least one optimal virtual EM triplet drive vector and at least one of the virtual EM triplet drive coils are generated using intermediate virtual drive x and y axes as plus and minus 45 degrees from the minimum virtual x-y-z vector and intermediate virtual drive z axis as orthogonal to the intermediate virtual drive x and y axis; the optimal virtual x, y, and z axis are then generated by pivoting the intermediate x, y, and z axes by moving the intermediate z axis 45 degrees about the maximum virtual x-y-z vector.

Such a system can be enhanced wherein the display further displays P-wave or other cardiac waveform changes over time in combination with the location of the medical device such as a guide wire or stylet tip in relationship to the subject's heart.

Such a system can be extended by further including (i) an x-axis tilt meter and y-axis tilt meter which uses gravity to measure the x-axis and y-axis tilt from true vertical; and (2) a computer to calculate and display the location of the medical device tip as height, width, and depth of the sensor coil corrected for the tilt the geometric array. Such a system can be enhanced wherein the geometric array and sensor coil connected to the display via a wireless interface.

Such system can be extended by further including an electrocardiogram (ECG) operably associated with the geometric array with a display to show the subject's ECG signal over time; or by further including an electroencephalogram (EEG) operably associated with the geometric array with a display to show the subject's EEG signal over time.

An aspect of the present developments can include a method for locating a medical device in a subject, including: (a) providing a system as presented herein; (b) inserting and positioning the medical device tip associated with a functional and sterile medical device into the subject; and (c) recording or monitoring the output of the display to locate the medical device tip in the subject.

Such a method can be extended wherein the method further includes the use of at least one selected an electrocardiogram (ECG), an electroencephalogram (EEG), an x-ray machine, an computer assisted tomography (CAT) machine, a positron emission tomography (PET) machine, an endoscope, or an ultrasound imaging device or composition.

METHODS, COMPUTER SYSTEMS AND SOFTWARE

An aspect of the present developments can include methods, computer systems and software, provided as programming code or instructions on computer readable media or hardware or networks or computer systems, for generating virtual electromagnetic (EM) triplet drive coils for generating data corresponding to the location coordinates for a sensor coil, including:

(a) electronically providing triplets of drive current values generated from at least three EM drive coils of the EM triplet drive coils in detectable proximity to the EM sensor at regular time intervals to provide one or more paired magnetic drive coil vector values generated at angles from 0 to 90 degrees without and from -90 to 0 degrees with phase inversion;

(b) electronically providing angle values of maximum or minimum EM sensor coil responses generated from the EM triplet drive coils within the x-y plane using paired x-y coils' programmable current drive sweeping a range from 0 to 90 degrees without and from -90 to 0 degrees with phase inversion;

(c) electronically computing at least one set of optimal virtual drive x and y axes as values corresponding to plus and minus 45 degrees from the maximum or the minimum sensor coil response;

(d) electronically computing an optimal virtual drive z axis orthogonal to the plane of the optimal virtual drive x and y axes to provide at least one optimal set of virtual EM triplet drive axes for at least one of the EM triplet drive coils.

Such a method can be extended wherein (a) the triplets of drive current values selected at regular time intervals arc provided as (i) paired magnetic drive coil vector values at angles from 0 to 90 degrees in an x-y plane added together with coil vectors of the z-axis from 0 to 90 degrees from the x-y plane; and (ii) as phase inversion of one or more paired magnetic drive coil vector values at angles from -90 to 0 degrees in an x-y plane added together with coil vectors of the z-axis from -90 to 0 degrees from the x-y plane; (b) the angle value of maximum sensor coil response within the x-y plane for both 0 to 90 and -90 to 0 degrees is determined as the intermediate virtual maximum x-y axis and this intermediate virtual maximum x-y axis added to the z-axis swept from 0 to 90 and -90 to 0 degrees to determine at least one maximum virtual x-y-z vector for at least one of the corresponding EM triplet drive coils; (c) the angle value of minimum sensor coil response within the x-y plane for both 0 to 90 and -90 to 0 degrees is determined as the intermediate virtual minimum x-y axis, and this intermediate virtual minimum x-y axis is added to the z-axis swept from 0 to 90 and -90 to 0 degrees to determine at least one minimum virtual x-y-z vector for at least one of the corresponding EM triplet drive coils; and (d) the at least one optimal virtual EM triplet drive axes for at least one of the EM triplet drive coils are calculated using plane defined by the maximum virtual x-y-z vector and minimum x-y-z vector wherein the optimal virtual drive x and y axes are plus and minus 45 degrees from the minimum virtual x-y-z vector and the optimal virtual drive z axis as orthogonal to the optimal virtual drive x and y axes.

Fig. 1 is an overview schematic diagram of an implementation of the present developments. A control box **20** contains a touch screen display **22** with a computer control and data processing. The triangular patient drive block **36** is connected via power and communications cable **26** to the control box **20.** The drive and sensor electronics **28** may be located in the block **36** and provide drive coil control and sensor coil demodulation/amplification. In the three corners of the block **36** are mounted each triplet drive coil set **24 (24a, 24b, 24c).** Alternatively, each coil set **24** could be mounted as feet protruding from the bottom of the block **36.** The sensor coil **30** is built onto a small diameter biocompatible cable **32** which may be inserted into a medical device such as a catheter to be placed in the patient. The sensor signal is connected back to the control box **20** with a two-wire cable **34.** Figure 3 provides a detailed view of a sensor coil **30.** Sensor coil performance can be improved by winding the coil about a ferromagnetic wire in the tip of the cable **48.** The ferromagnetic material should be used for the length of the sensor coil **30** and may be followed by non-ferrous material. Two fine wires **50** from the sensor coil **30** are attached or associated (e.g., glued or wrapped) and sealed **49** down the length of the cable **48.**

As presented, e.g., in Fig. 8, an aspect of this device may include continuous display at the position of the sensor coil **30** placed in the tip of a medical device as the medical device moves through the patient's tissue. The patient drive block **36, 60,** is placed over the patient's body where the medical device will be targeted (e.g., over the chest preferably aligned with the sternal notch if the medical device will be placed in the area of the heart).

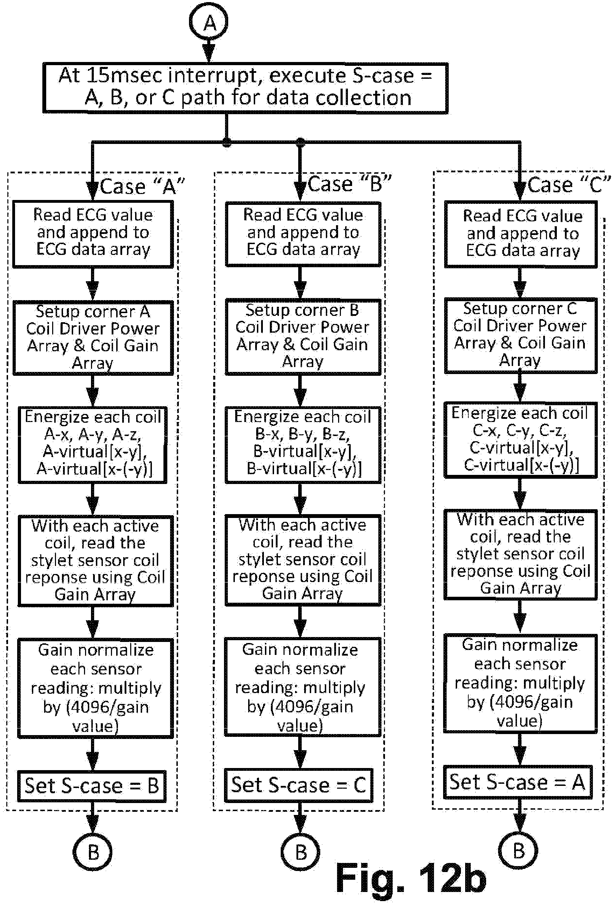

As presented, e.g., in Fig. 1 and Fig. 7, respectively, the sensor coil cable **34, 54** and patient drive block cable **26, 56** arc connected to a user control box **20.** The user control box **20** contains a computer **68** (Fig. 9) which sequentially drives each driver coil **38, 40, 42** (Fig. 2) on each axis in every corner **24a, 24b, 24c** of the patient drive block **36, 60.** The coil driver creates magnetic drive vectors as shown in Figures 4, 5, or 6 - where Figure 4 represents normal drive and Figures 5 and 6 represent virtual drive. The single board computer **68** (Fig. 9) measures the demodulated output signal from the sensor coil **30** for each sequentially driven coil **24** (Fig. 1). The single board computer **68** (Fig. 9) continuously adjusts the gain of each output signal using a programmable gain stage in the demodulator electronics and scales all the sensor data to be gain normalized (see Figure 12b). Here, gain normalized means that if a measured response is value "a" collected at a programmable gain of "4.00" times, then the normalized resultant value is "a" divided by 4.00. A non-limiting example of an alternative gain normalizing method is to multiply each value "a" by 4096 / gain, e.g. "a" x 4096/4.00. The programmable gains that can be used as a non-limiting example are 1.00, 2.00, 4.00, 8.00, 16.00 and 32.00 plus there can be a final gain stage that is selectable for 1.00 or 1.41 (equivalent to 1/√2). Examples of a resultant set of programmable gains arc 1.00, 1.41, 2.00, 2.82, 4.00, 5.64, 8.00, 11.28, 16.00, 22.56 and 32.00.

From the normalized response data, the single board computer **68** (Fig. 9) compares the sum of the squares of the sensor's normal response to the sum of the squares of the sensor's virtual response. Whichever sum is greater or has the stronger response can be used by the computer **68** to calculate the sensor coil **30** location using trilateration which is the calculation of the intersection of three spheres where cach sphere is defined as the radial distance of the sensor coil **30** from each x-y-z driver coil set **24a, 24b, 24c** (Fig. 1).

From each corner, the radial distance can be defined as a constant divided by the 6th root of the sum of the squares of the x. y, and z measured normalized response. Here the constant, k, is a calibration constant reflecting the strength of each drive coil **38, 40, 42** (Fig. 2) and the sensitivity of the sensor coil **30** (Fig. 1). In one possible aspect, a calibration constant could be generated during manufacturing for each of the drive coils and then stored as calibration constants for each coil in non-volatile memory of the patient drive block. By trilateration, the radial distance equation for each corner is:

$$r = k \,/\, \sqrt[6]{((x^2 + y^2 + z^2))}$$

The sensor coil location is then calculated from three equations for the three corners of plate **36:**

$$r_1^2 = k^2 \,/\, \sqrt[3]{((x_1^2 + y_1^2 + z_1^2))}$$

$$r_2^2 = k^2 \,/\, \sqrt[3]{((x_2 - d)^2 + y_2^2 + z_2^2)}$$

$$r_3^2 = k^2 \,/\, \sqrt[3]{(x_3^2 + (y_3 - d)^2 + z_3^2)}$$

Here **d** is the distance of each coil from the other and k is the calibration constant which scales the result into meaningful units of distance, e.g. centimeters or inches. In this non-limiting example, the coils on the patient drive plate or block **36, 60** (Figs. 1 and 7) arc arranged in a right triangle, with two sides of equal length, d, as reflected in the 2nd and 3rd equations above, just on different axis, x or y. The solution to these equations yielding the sensor coil location is:

$$x_S = (r_1^2 - r_2^2 + d^2) \,/\, 2d$$

$$y_S = (r_1^2 - r_3^2 + d^2) \,/\, 2d$$

$$z_S = +/- \sqrt{(r_1^2 - x_S^2 - y_S^2)}$$

Here the solution for z (vertical axis) is assumed to be negative as the sensor coil **30** cannot be above the plane of the patient drive block **36, 60** (Figs. 1 and 7) unless it is outside the patient.

While the above reflects one non-limiting approach to locating the sensor coil **30** (Fig. 1), it is not the only or optimal for coil location. Specifically when the sensor coil **30** is nearly perpendicular to a driver coil axis the sensor coil response approaches zero; therefore a "virtual axis" drive system provides for the optimal sensor coil response. In this approach the coil driver circuit is designed to selectively drive, within a triplet set, pairs of coils, e.g. **38** and **42** (Fig. 2), together at the same frequency and amplitude with an additional driver control to selectively invert the phase of one coil in the pair. This paired coil drive of x-y coils creates the 1st virtual axis at forty-five degrees from the original x and y axes. The paired coil drive is then operated with x-(-y) which drives the x-coil together with inverted-phase y-coil and this creates the 2nd virtual axis at minus forty-five degrees from the original x and y axes. The result is two orthogonal virtual magnetic vectors as shown by the dashed lines in Figure 5 for x-y paired coil drive or in Figure 6 for y-z paired coil drive. For paired drive, the single board computer **68** (Fig. 9) sequentially drives the x-y pair, x-(-y) pair, and z axis for each x-y-z coil set **24a, 24b, 24c** (Figs. 1 and 7). Here, "(-y)" means inverted phase on y axis drive. The measured sensor coil response for paired-coil drive must be scaled down by 1.4142 because of vector summing of the two coils driven together. Alternatively, the coil-driver power could be scaled down by 0.7071 in hardware when driving pairs so that the vector sum of two coils equals the magnetic vector of a single coil drive. The single board computer **68** measures the sensor coil response for each corner in normal drive and paired drive, and then selects the strongest signal from each corner comparing the sum of $x^2$, $y^2$, and $z^2$ normal coil drive response to the sum of $(x-y)^2$, $(x-(-y))^2$, and $z^2$ paired coil drive response. The strongest signal from each corner is used to calculate the location of the sensor coil **30** using the trilateration method described in the above equations. This example illustrates paired x-y coil drive; and by logical extension this may also apply to x-z, or y-z paired drive. An objective in some implementations of these developments may include improving the accuracy of horizontal (x-y) location; therefore the paired x-y drive can be preferred over x-z or y-z.

An improvement of the paired-coil drive can be to add programmable (DAC) power control to the coil drivers on the drive coil drive electronics board **28** (Fig. 11). Here, the single board computer **68** (Fig. 9) has the capability to select pairs of drive current power settings which steer the virtual axis of the paired coils from 0 to 90 degrees. Inversion of one of the coil drivers in the pair provides the capability for virtual axis from -90 to 0 degrees. In this design, the single board computer **68** selects pairs of power settings output to the x-y paired coil driver to sweep the virtual drive axis from 0 to 90 degrees while recording the sensor coil response and this process is repeated with the y coil driver phase inverted to sweep from -90 to 0 degrees. The angle of the virtual axis when the sensor coil response data is maximum indicates the sensor coil **30** (Fig. 1) is parallel to the virtual axis and the angle of the virtual axis when the sensor coil response data is minimum indicates the sensor coil **30** is perpendicular to the virtual axis. Here the maximum angle and minimum angle are orthogonal (perpendicular). The single board computer **68** (Fig. 9) calculates the optimum virtual x axis at forty-five degrees from the measured angle for maximum (or minimum) response and calculates the optimum virtual y axis as an angle orthogonal to the virtual x axis. As all x-y-z coil sets **24a, 24b, 24c** are mechanically aligned the solution for best virtual axes in one corner applies to all corners in the patient drive block **36, 60** (Figs.1 and 7). The single board computer **68** (Fig. 9) measures the sensor coil response for all corners using these optimum virtual x axis, optimum virtual y axis plus normal z axis. The sum of (virtual x)$^2$, (virtual y)$^2$, and $z^2$ sensor coil responses for each corner is used to calculate the position of the sensor coil **30** (Fig. 1) using the trilateration method described in the above equations. This example illustrates paired x-y coil drive; and by logical extension this also applies to x-z, or y-z paired drive; however, the preference in this development is to improve accuracy of horizontal location and thus use paired x-y drive.

An alternative, non-limiting, method for finding the optimum virtual x and virtual y axis in the programmable pair-coil drive above is to use successive approximation instead of sweeping 0 to 90 degrees. In this approach, the single board computer **68** (Fig. 9) has the capability to select pairs of drive current power settings which steer the virtual axis from -90 to +90 degrees. The single board computer first tests the sensor coil response to the paired coils at virtual axes +45 and -45 degrees and selects the virtual axis with the stronger response. Using the stronger axis, the computer then tests the sensor coil response to paired coils at +22.5 and -22.5 degrees from the current virtual axis and selects the virtual axis with the stronger response. This process continues for +/-11.25 degrees, +/-5.625 degrees, until the limits of drive power resolution are reached. The resulting virtual axis is the axis of maximum response. The single board computer **68** calculates the optimum virtual x axis at forty-five degrees from the measured angle for maximum response and calculates the optimum virtual y axis as an angle orthogonal to the virtual x axis.

A selection of the best set of virtual axes can be accomplished with a triplet-coil drive scheme, with programmable power control to the coil drivers for each axis, and with the driver control to selectively invert the phase of any coil in the triplet x-y-z coil sets **24a, 24b, 24c** (see Figure 15). Here, the single board computer **68** (Fig. 9) has the capability to select pairs of drive current power settings which steer the x-y virtual axis of the paired coils from 0 to 90 degrees. Inversion of one of the coil drivers in the pair provides the capability for x-(-y) virtual axis from -90 to 0 degrees. With the addition of the third coil drive and inversion the single board computer **68** can sweep the virtual axis 0 to 90 and -90 to 0 degrees in z range. The microcomputer selects pairs of current settings output to the x-y paired coil driver to sweep the virtual drive axis from 0 to 90 degrees while recording the sensor coil response and this process is repeated with the y coil driver phase inverted to sweep from -90 to 0 degrees. The angle of the x-y virtual axis when the sensor coil response data is maximum indicates the sensor coil **30** (Fig. 1) is parallel for the x-y plane. The single board computer **68** then sets this x-y axis and sweeps the z axis drive from -90 to 0 and 0 to 90 degrees while recording the sensor coil response. The polan angle of the x-y-z virtual axis when the sensor coil response data is at maximum indicates the sensor coil **30** is parallel to this virtual x-y-z axis. The single board computer **68** then repeats this process to find the minimum sensor coil response sweeping x, y, and z axes. The polar angle of the x-y-z virtual axis when the sensor coil response data is minimum indicates the sensor coil **30** is perpendicular to this virtual x-y-z axis. These two vectors, virtual minimum and virtual maximum, define a plane intersecting the sensor coil **30.** For optimum response, the single board computer **68** calculates a virtual x axis 45 degrees between the maximum and minimum vectors, then calculates the virtual y axis as 90 degrees from the virtual x in the plane defined previously. Here virtual z axis is defined as orthogonal to the plane of virtual minimum and virtual maximum vectors. The single board computer **68** then tilts the virtual z axis and the plane of virtual x axis and virtual y axis 45 degrees toward the virtual minimum vector, and the result is the optimal virtual axis set which maximizes the sensor coil response. As all x-y-z coil sets **24a, 24b, 24c** are mechanically aligned the solution for best virtual axes in one corner applies to all corners in the driver array. The single board computer **68** measures the sensor coil response for all corners using these optimum virtual x, y, and z axes. The sum of (virtual x)$^2$, (virtual y)$^2$, and (virtual z)$^2$ sensor coil responses for each corncr may be used to calculate the sensor coil **30** using the trilateration method described in the above equations. One method to maintain the optimum x-y-z axis over time is to continuously test the sensor coil response to small deviations (offset angle) from the optimum axis (see Fig 16c). Here, the single board computer **68** compares the sum of (virtual x)$^2$, (virtual y)$^2$, and (virtual z)$^2$ sensor coil responses for the current virtual axis to the sum for virtual axis plus offset angle and the sum for virtual axis minus offset angle. The computer **68** then selects the axis with the largest summed response - this becomes the new optimum x-y-z axis and the process continues to iterate testing small deviations over time.

The single board computer **68** may then graphically display the sensor coil position on the display **22** of the control box **20**. The position is continuously updated adding onto the previous graphical data to create a track or path of the sensor coil **30** over time. The user interface of the single board computer **68** allows the user to clear the recorded track or to save the recorded track to non-volatile memory. Touchscreens have been described; however keyboard or other data input, or user interface options may be used.

The construction details above for the control box **20** (Figs. 1, 7) may provide for a tethered device with the display/control separate from the patient block **36, 60** (Figs. 1 and 7). However, an alternative construction would be to build a device or system in which the patient block **36, 60** is battery-powered and connected wirelessly to the control box **20**. In another variation, the control box **20** could be integrated into or as part of the patient block and placed on the patient chest or other locations to track medical device position. Wireless and/or wired connections are thus optionally available for the connections of the drive coil sets to the control or system components for the driving thereof; as well as for the connections of the sensor coil to the control or system components for measuring or receiving the response signals of the sensor coil.

An alternative construction would be to use four or more drive coils **24** oriented as a square, rectangle, pentagon, circle, oval, geometric, or any other suitable shape, in or as the patient drive block **36, 60** (Figs. 1, 7).

An alternative construction (Fig. 7) is to optionally incorporate electrocardiograph (ECG) monitoring in-

to the medical device location system to facilitate placement of the medical device with sensor coil **30** in close proximity to the heart. Here the patient drive block **60** may be modified with one or more ECG pads **64** and ECG lead wires **62** which attach to the patient's chest and the third ECG lead is provided by a conductive wire **52** added in or otherwise made part of the core of the guide wire or stylet sensor coil **30**.

An ECG amplifier can be added to the main interface board **70** (Fig. 9), and the single board computer **68** may then present the ECG on the display **22** as the medical device such as a catheter is advanced within the patient's or subject's body. The user can observe changes in the P-wave or other wave elements of the ECG as the medical dcvice/catheter reaches the heart. Ideally, the single board computer **68** could use a waveform analysis to assist the user in recognizing changes occurring to the P-wave or other waveforms.

A component to this design may include connecting the signals from the sensor coil **30** and ECG **52** to the user control box **20**. This is complicated in practice by covering the entire patient and patient block **60** with sterile drapes for insertion of the patient's medical device/catheter. In this design, a miniature stereo phone plug or similar could be used to pierce a plastic bag and connect to cable **34, 54** a pigtail from the user control box **20**.

Methods, devices and systems can thus be provided for one or both of two- or three-dimensional location of the disposition of a sensor coil in a subject including: an array of electromagnetic drive coil sets, each set having two or three dimensionally oriented drive coils; a sensor coil being electromagnetically communicative with the array of electromagnetic drive coil sets; and, a system controller communicative with and adapted to energize one or more of the electromagnetic coils in the array of electromagnetic drive coil sets, the energizing of the one or more of the electromagnetic coils including one or more of energizing the coils singly, or in pairs of x-y and y-z or x-z coils while measuring the response of the sensor coil; whereby the system uses the measurements of the responses of the sensor coil to calculate the location and orientation of the sensor coil relative to said drive coil sets.

This may include two- and/or three-dimensional location of a catheter in tissue using an array of x-y or x-y-z oriented electromagnetic coils, where a sensor coil can be associated with one or more catheter tips, and where the system controller can energize one or more external coils, such as but not limited to, pairs of x- y and y-z or x-z coils while measuring the response of the sensor coil; the system can use these sensor coil measurements to calculate the position and orientation of the catheter tip, and in some implementations, the system controller can graphically display the catheter tip position, depth and/or orientation, e.g., but not limited to, over time.

The invention is limited only by the scope of the claims appended hereto.

**Claims**

1. A medical device locating system for determining disposition of a sensor coil (30) in a subject, said system comprising:

   a. an array of three or more triplet drive coil sets (24) arranged in a block which is configured to be placed on a patient's chest, each drive coil set including at least three discrete drive coils (38, 40, 42), each of the discrete drive coils being electromagnetic coils;
   b. at least one sensor coil adapted to provide one or more sensor coil response signals;
   c. a first system component configured to provide AC drive signals energizing said discrete drive coils, wherein the provision of drive signals includes one or both:

      (i) sequentially driving one or a pair or a triplet of said discrete drive coils within a triplet drive coil set; and
      (ii) selectively providing phase inversion of the drive signal to any one, two or three of said discrete drive coils within a triplet drive coil set;

   d. a second system component for measuring resulting one or more sensor coil response signals, including one or more of sequential single, paired or triplet sensor coil response signals;
   e. an electrocardiogram (ECG) operably associated with said triplet drive coil array wherein one or more ECG reference leads are configured to be placed on said subject and a ECG signal lead is provided by a conductive core wire supporting said sensor coil; and
   f. a computing component configured to calculate sensor coil disposition in the subject relative to said triplet drive coil sets, **characterized in that** the system further comprises:
   g. a display (22) configured to show the disposition of said sensor coil in said subject relative to the array of said triplet drive coil sets in combination with the ECG signal of the subject over time, wherein said display is configured to display a P-wave or other wave changes of the ECG of the subject in combination with the disposition of said sensor coil and wherein the computing component is further configured to use a waveform analysis to assist a user in recognizing changes occurring to the P-wave of the ECG or wave elements of the ECG signal,
   the system further comprising one or more of a catheter, a guide wire and a stylet with said sensor coil being disposed in physical association therewith, and further comprising an integrated conductive ECG lead associated with the sensor coil, the conductive ECG lead providing an electrical ECG signal, wherein the display (22) is configured to display the P-wave changes or other waveform changes so as to indicate a proximity of the catheter, stylet or guide wire relative to the heart of the subject.

2. A system according to one of the previous claims, wherein the sensor coil comprises an insulated wire wound around a ferrous core wire in one or more layers.

3. A system according to one of the previous claims, wherein said discrete drive coils within one or more of the triplet drive coil sets are disposed in an orthogonal array.

4. A system according to one of the previous claims, wherein said discrete drive coils are arranged in perpendicular axes relative to each other in an x-y-z array within each of said triplet drive coil sets; each x, y and z axis of each of said triplet drive coil sets being arranged in parallel with the respective x, y and z axes of all other of said triplet drive coil sets in said array.

5. A system according to one of the previous claims, wherein the sensor coil comprises a single coil.

6. A system according to one of the previous claims, wherein the computing component comprises a processor.

7. A system according to one of the previous claims, wherein the computing component is configured to calculate the sensor coil disposition in the subject relative to said triplet drive coil sets from one or more measured resulting sensor coil response signals.

8. A system according to one of the previous claims, wherein the provision of drive signals includes selectively providing phase inversion of the drive signal to selectively invert the phase of one coil in the triplet drive coil set.

**Patentansprüche**

1. Medizingerätlokalisierungssystem zum Bestimmen der Position einer Sensorspule (30) in einer Person, wobei das System Folgendes umfasst:

   a) eine Anordnung von drei oder mehr Dreiergruppenantriebsspulen-Sets (24), die in einem Block angeordnet sind, der dazu konfiguriert ist, auf der Brust eines Patienten platziert zu werden, wobei jedes Antriebsspulen-Set mindestens drei separate Antriebsspulen (38, 40, 42) beinhaltet, wobei jede der separaten Antriebsspulen eine elektromagnetische Spule ist;

b) mindestens eine Sensorspule, die dazu ausgelegt ist, ein oder mehrere Sensorspulenreaktionssignale bereitzustellen;

c) eine erste Systemkomponente, die dazu konfiguriert ist, Wechselstromantriebssignale, die Energie an die separaten Antriebsspulen liefern, bereitzustellen, wobei das Bereitstellen von Antriebssignalen beinhaltet:

(i) sukzessives Antreiben einer oder eines Paares oder einer Dreiergruppe der separaten Antriebsspulen innerhalb eines Dreiergruppenantriebsspulen-Sets; und/oder

(ii) selektives Bereitstellen einer Phasenumkehr des Antriebssignals an eine, zwei oder drei der separaten Antriebsspulen innerhalb eines Dreiergruppenantriebsspulen-Sets;

d) eine zweite Systemkomponente zum Messen von einem oder mehreren resultierenden Sensorspulenreaktionssignalen, welche ein oder mehrere sukzessive Einzel-, Paar- oder Dreiergruppen-Sensorspulenreaktionssignale beinhalten;

e) ein Elektrokardiogramm (EKG), das funktionsbereit mit der Dreiergruppenantriebsspulen-Anordnung verbunden ist, wobei ein oder mehrere EKG-Referenzableitungselektroden dazu konfiguriert sind, auf der Person platziert zu werden, und wobei eine EKG-Signalableitungselektrode durch einen Draht mit leitfähigem Kern, der die Sensorspule trägt, bereitgestellt ist; und

f) eine Rechenkomponente, die dazu konfiguriert ist, die Sensorspulenposition in der Person relativ zu den Dreiergruppenantriebsspulen-Sets zu berechnen, **dadurch gekennzeichnet, dass** das System weiter Folgendes umfasst:

g) eine Anzeige (22), die dazu konfiguriert ist, die Position der Sensorspule in der Person relativ zu der Anordnung der Dreiergruppenantriebsspulen-Sets in Kombination mit dem EKG-Signal der Person über die Zeit darzustellen, wobei die Anzeige dazu konfiguriert ist, Veränderungen der P-Welle oder anderer Wellen des EKG der Person in Kombination mit der Position der Sensorspule anzuzeigen, und wobei die Rechenkomponente weiter dazu konfiguriert ist, eine Wellenformanalyse zu verwenden, um einen Benutzer bei der Erkennung von Veränderungen, die bei der P-Welle des EKG oder Wellenelementen des EKG-Signals auftreten, zu unterstützen,

wobei das System weiter einen Katheter, einen Führungsdraht und/oder ein Stilett umfasst, wobei die Sensorspule in physischer Verbindung damit angeordnet ist, und weiter eine integrierte leitfähige EKG-Ableitungselektrode umfasst, die mit der Sensorspule verbunden ist, wobei die leifähige EKG-Ableitungselektrode ein elektrisches EKG-Signal bereitstellt, wobei die Anzeige (22) dazu konfiguriert ist, die P-Wellen-Veränderungen oder andere Wellenformveränderungen anzuzeigen, um eine Nähe des Katheters, Stiletts oder Führungsdrahts relativ zum Herzen der Person anzugeben.

2. System nach einem der vorstehenden Ansprüche, wobei die Sensorspule einen um einen eisenhaltigen Kern gewickelten isolierten Draht in einer oder mehreren Schichten umfasst.

3. System nach einem der vorstehenden Ansprüche, wobei die separaten Antriebsspulen innerhalb eines oder mehrerer der Dreiergruppenantriebsspulen-Sets in einer orthogonalen Anordnung angeordnet sind.

4. System nach einem der vorstehenden Ansprüche, wobei die separaten Antriebsspulen in senkrechten Achsen relativ zueinander in einer x-y-z-Anordnung innerhalb jedes der Dreiergruppenantriebsspulen-Sets angeordnet sind; wobei jede x-, y- und z-Achse jedes der Dreiergruppenantriebsspulen-Sets parallel zu der jeweiligen x-, y- und z-Achse aller anderen der Dreiergruppenantriebsspulen-Sets in der Anordnung angeordnet ist.

5. System nach einem der vorstehenden Ansprüche, wobei die Sensorspule eine einzelne Spule umfasst.

6. System nach einem der vorstehenden Ansprüche, wobei die Rechenkomponente einen Prozessor umfasst.

7. System nach einem der vorstehenden Ansprüche, wobei die Rechenkomponente dazu konfiguriert ist, die Sensorspulenposition in der Person relativ zu den Dreiergruppenantriebsspulen-Sets aus einem oder mehreren gemessenen resultierenden Sensorspulenreaktionssignalen zu berechnen.

8. System nach einem der vorstehenden Ansprüche, wobei die Bereitstellung von Antriebssignalen das selektive Bereitstellen einer Phasenumkehr des Antriebssignals beinhaltet, um die Phase einer Spule in dem Dreiergruppenantriebsspulen-Set selektiv umzukehren.

**Revendications**

1. Système de localisation de dispositif médical destiné à déterminer la disposition d'une bobine de détection (30) à l'intérieur d'un sujet, ledit système comprenant :

a. un réseau de trois ensembles de triplets de bobines d'excitation (24) ou plus agencé en un bloc qui est configuré pour être placé sur le thorax d'un patient, chaque ensemble de bobines d'excitation comprenant au moins trois bobines d'excitation discrètes (38, 40, 42), chacune des bobines d'excitation discrètes étant des bobines électromagnétiques ;
b. au moins une bobine de détection adaptée à fournir un ou plusieurs signaux de réponse de bobine de détection ;
c. un premier composant de système configuré pour fournir des signaux d'excitation AC mettant sous tension lesdites bobines d'excitation discrètes, dans lequel la fourniture des signaux d'excitation inclut l'un ou les deux de :

(i) l'excitation séquentielle d'une ou d'une paire ou d'un triplet desdites bobines d'excitation discrètes à l'intérieur d'un ensemble de triplets de bobines d'excitation ; et
(ii) la fourniture sélective d'une inversion de phase du signal d'excitation à une, deux ou trois desdites bobines d'excitation discrètes à l'intérieur d'un ensemble de triplets de bobines d'excitation ;

d. un second composant de système pour mesurer un ou plusieurs signaux de réponse de bobine de détection, y comprenant un ou plusieurs signaux de réponse de bobines de détection seules, en paire ou en triplet ;
e. un électrocardiogramme (ECG) fonctionnellement associé au dit réseau de triplets de bobines d'excitation dans lequel une ou plusieurs dérivations de référence d'ECG sont configurées pour être placées sur le sujet et une dérivation de signal d'ECG est fournie par un fil d'âme conducteur supportant ladite bobine de détection ; et
f. un composant de calcul configuré pour calculer la disposition d'une bobine de détection dans le sujet par rapport auxdits ensembles de triplets de bobines d'excitation, **caractérisé en ce que** le système comprend en outre :
g. un affichage (22) configuré pour présenter la disposition de ladite bobine de détection dans ledit sujet par rapport au réseau desdits ensembles de triplets de bobines d'excitation en combinaison avec le signal d'ECG du sujet dans le temps, dans lequel ledit affichage est configuré pour afficher une onde P ou d'autres modifications d'ondes de l'ECG du sujet en combinaison avec la disposition de ladite bobine de détection et dans lequel le composant de calcul est en outre configuré pour utiliser une analyse de forme d'onde pour aider un utilisateur à reconnaître les modifications se produisant sur l'onde P de l'ECG ou les éléments d'onde du signal d'ECG, le système comprenant en outre un ou plusieurs d'un cathéter, d'un fil-guide et d'un stylet, ladite bobine de détection étant disposée en association physique avec celui-ci, et comprenant en outre une dérivation d'ECG conductrice intégrée associée à ladite bobine de détection, la dérivation d'ECG conductrice fournissant un signal électrique d'ECG, dans lequel l'affichage (22) est configuré pour afficher les modifications de l'onde P ou d'autres modifications de formes d'onde pour indiquer une proximité du cathéter, du stylet ou du fil-guide par rapport au cœur du sujet.

2. Système selon l'une des revendications précédentes, dans lequel la bobine de détection comprend un fil isolé enroulé autour d'un fil d'âme ferreux en une ou plusieurs couches.

3. Système selon l'une des revendications précédentes, dans lequel lesdites bobines d'excitation discrètes à l'intérieur d'un ou plusieurs des ensembles de triplets de bobines d'excitation sont disposées en un réseau orthogonal.

4. Système selon l'une quelconque des revendications précédentes, dans lequel lesdites bobines d'excitation discrètes sont agencées selon des axes perpendiculaires par les uns par rapport aux autres dans un réseau x-y-z à l'intérieur de chacun desdits ensembles de triplets de bobines d'excitation ; chaque axe x, y et z de chacun desdits ensembles de triplets de bobines d'excitation étant agencé parallèle aux axes x, y et z respectifs de tous les autres desdits ensembles de triplets de bobines d'excitation dans ledit réseau.

5. Système selon l'une quelconque des revendications précédentes, dans lequel la bobine de détection comprend une bobine unique.

6. Système selon l'une quelconque des revendications précédentes, dans lequel le composant de calcul comprend un processeur.

7. Système selon l'une quelconque des revendications précédentes, dans lequel le composant de calcul est configuré pour calculer la disposition de la bobine de détection à l'intérieur du sujet par rapport auxdits ensembles de triplets de bobines d'excitation à partir d'un ou plusieurs signaux de réponse de bobine de détection résultants.

8. Système selon l'une quelconque des revendications précédentes, dans lequel la fourniture de signaux d'excitation inclut la fourniture sélective d'une inversion de phase du signal d'excitation pour sélectivement inverser la phase d'une bobine dans l'ensemble de triplets de bobines d'excitation.

**Fig. 1**

**Fig. 2**

EP 3 165 156 B1

**Fig. 3**

**Fig. 4**

**Fig. 5**

**Fig. 6**

EP 3 165 156 B1

**Fig. 7**

EP 3 165 156 B1

**Fig. 8**

EP 3 165 156 B1

**Fig. 9**

Fig. 10

**Fig. 11**

- Patient Drive Coil Block Power → Voltage Regulators → To all Patient Drive Coil Block Circuits
- Flash Memory for Drive Coil Calibration Data & Board ID
- Supply Voltage & Drive Current Monitor
- Power Level A DAC → Coil Drive A Power Amp → Multi-plexer A
- Patient Drive Block Status and Control
- 9 Drive Coils Array
- Patient Drive Block Oscillator
- Polarity Inversion Switch → Power Level B DAC → Coil Drive B Power Amp → Multi-plexer B

Patient Isolated Section

- Patient Drive Block ECG Out
- LA ECG Plug
- LL ECG Plug

EP 3 165 156 B1

```
┌─────────────────────────┐
│   Pet main watchdog     │
└─────────────────────────┘
             │
             ▼
┌─────────────────────────┐
│  Test ECG status for    │
│  lead connections to    │
│  LA, LL and Stylet      │
└─────────────────────────┘
             │
             ▼
          ╱──────╲                                    ┌──────────────────────────────┐
         ╱ Lead-off ╲         Yes                     │  Display Advisory Message:   │
        ╱ condtion    ╲──────────────────────────────▶│     "ECG Lead Off"           │
        ╲ detected?   ╱                                └──────────────────────────────┘
         ╲           ╱                                             │
          ╲─────────╱                                             │
             │ No                                                 │
             │◀────────────────────────────────────────────────────┘
             ▼
          ╱──────╲                                    ┌──────────────────────────────┐
         ╱ Stylet  ╲           Yes                    │  Display Advisory Message:   │
        ╱ not        ╲─────────────────────────────── ▶│     "No Stylet"              │
        ╲ Connected? ╱                                └──────────────────────────────┘
         ╲          ╱                                              │
          ╲────────╱                                              │
             │ No                                                 │
             │◀────────────────────────────────────────────────────┘
             ▼
           ( A )
```

# Fig. 12a

(A)

At 15msec interrupt, execute S-case = A, B, or C path for data collection

**Case "A"**

Read ECG value and append to ECG data array

Setup corner A Coil Driver Power Array & Coil Gain Array

Energize each coil A-x, A-y, A-z, A-virtual[x-y], A-virtual[x-(-y)]

With each active coil, read the stylet sensor coil reponse using Coil Gain Array

Gain normalize each sensor reading: multiply by (4096/gain value)

Set S-case = B

(B)

**Case "B"**

Read ECG value and append to ECG data array

Setup corner B Coil Driver Power Array & Coil Gain Array

Energize each coil B-x, B-y, B-z, B-virtual[x-y], B-virtual[x-(-y)]

With each active coil, read the stylet sensor coil reponse using Coil Gain Array

Gain normalize each sensor reading: multiply by (4096/gain value)

Set S-case = C

(B)

**Case "C"**

Read ECG value and append to ECG data array

Setup corner C Coil Driver Power Array & Coil Gain Array

Energize each coil C-x, C-y, C-z, C-virtual[x-y], C-virtual[x-(-y)]

With each active coil, read the stylet sensor coil reponse using Coil Gain Array

Gain normalize each sensor reading: multiply by (4096/gain value)

Set S-case = A

(B)

# Fig. 12b

( B )

Append Sensor Results into
Sensor Data Results Boxcar
Array

ECG Lead
Off condition
detected? —— Yes —— ( C )

No

Output ECG data points
to display and buffer

Find max and min
readings in 8 slices of
1.5 seconds of buffered
ECG data [12 sec total].

Below
LowECGLimit? —— Yes —— Increment
ECG Gain

No

Divide ECG display range
by the average of 8
(max-min) values

Above
HighECGLimit? —— Yes —— Decrement
ECG Gain

No

**Fig. 12c**

( C )

C

Calculate sensor AGC for each coil, average 5 readings for each sensor responses

Below Low Sensor Limit? — Yes → Is Coil Drive at Low Power? — Yes → Set coil drive Array to High Power → D

Below Low Sensor Limit? — No ↓

Is Coil Drive at Low Power? — No ↓

Increment Gain Array for the coil

Above High Sensor Limit? — Yes → Decrement Gain Array for the coil

Above High Sensor Limit? — No ↓

Gain Array = Min Gain and Coil Drive at High Power? — No ← / — Yes → Set coil drive Array to Low Power → D

Scan for control button presses

D

**Fig. 12d**

D

$\geq$5 Readings for each corner? — No → E

Yes ↓

Boxcar Average last 5 readings for each sensor response

Is A-corner sum [$x^2+y^2$] > A-virtual-corner [$(x-y)^2+(x-(-y))^2$] ? — No → Use virtual axis for positon calculation

Yes ↓

Use Normal axis for position calculation → Calculate sensor coil position

Display sensor position as green circle

↓

E

# Fig. 12e

(E)

"Start"? ——No——→ "Snapshot ECG"? ——No——→

| Yes | Yes |

Display sensor coil position as "rabbit trail"

Snapshot 1 full ECG complex and append to display of Saved ECG images

Lead Off ? ——No——→

Yes

Snapshot 1 full ECG comples and display as 1st saved ECG image

Replace "Start" button on display with "Snapshot ECG" icon

"Finish"? ——No——→ (A)

Yes

Close Data Files & Stop Data Collection

# Fig. 12f

**16 kHz Oscillator**

To Sense Coil Demodulator

Computer Control Hi/Low Power 1 & 2

**Set Drive Power Hi/Low**

**Coil Driver Amp1**

**Multi-plexer 1**

Acorner x-Coil
Bcorner x-Coil
Ccorner x-Coil
Acorner z-Coil
Bcorner z-Coil
Ccorner z-Coil

**Set Drive Power Hi/Low**

**Coil Driver Amp2**

**Multi-plexer 2**

Acorner y-Coil
Bcorner y-Coil
Ccorner y-Coil

Computer Control Normal/Invert Amp 2

Computer Control Address Mux 1 & 2

**Fig. 13**

Fig. 14

EP 3 165 156 B1

Fig. 15

A

At 15msec interrupt, execute S-case = A, B, or C path for data collection

**Case "A"**

Read ECG value and append to ECG data array

Setup corner A Coil Driver Power Array & Coil Gain Array

Energize coil set A-virtual1[x-y-z], A-virtual2[x-y-z], A-virtual3[x-y-z], and virtual1(±offset1), virtual2(±offset2), virtual3(±offset3).

With each active coil, read the stylet sensor coil reponse using Coil Gain Array

Gain normalize each sensor reading: multiply by (4096/gain value)

Set S-case = B

B

**Case "B"**

Read ECG value and append to ECG data array

Setup corner A Coil Driver Power Array & Coil Gain Array

Energize coil set B-virtual1[x-y-z], B-virtual2[x-y-z], B-virtual3[x-y-z]

With each active coil, read the stylet sensor coil reponse using Coil Gain Array

Gain normalize each sensor reading: multiply by (4096/gain value)

Set S-case = C

B

**Case "C"**

Read ECG value and append to ECG data array

Setup corner A Coil Driver Power Array & Coil Gain Array

Energize coil set C-virtual1[x-y-z], C-virtual2[x-y-z], C-virtual3[x-y-z]

With each active coil, read the stylet sensor coil reponse using Coil Gain Array

Gain normalize each sensor reading: multiply by (4096/gain value)

Set S-case = A

B

# Fig. 16a

```
                        ( C )
                          |
                          v
        +---------------------------------------+
        |     Calculate sensor AGC for          |
        |     each coil, average 5              |
        |     readings for each sensor          |
        |     responses                         |
        +---------------------------------------+
                          |
                          v
                   / Below Low \        Yes
                  <  Sensor Limit? >---------+
                   \            /            |
                      No                     |
                       |                     v
                       |          +------------------------+
                       |<---------|   Increment Gain       |
                       |          |   Array for the coil    |
                       |          +------------------------+
                       v
                   / Above High \        Yes
                  <  Sensor  Limit? >---------+
                   \            /             |
                      No                      |
                       |                      v
                       |           +------------------------+
                       |<----------|   Decrement Gain       |
                       |           |   Array for the coil    |
                       |           +------------------------+
                       v
        +---------------------------+
        |   Scan for control        |
        |   button presses          |
        +---------------------------+
                       |
                       v
                     ( D )
```

# Fig. 16b

D

≥5 Readings for each corner? — No → E

Yes ↓

Boxcar Average last 5 readings for each sensor response

↓

Using trilateration calculate sensor coil position

↓

Display sensor position as green circle

↓

Boxcar Average last 5 "offset" readings for "A" sensor response

↓

Test for strongest signal, find-max of:

sum a: $A\text{-}virtual1[+offset1]^2 + A\text{-}virtual2[+offset2]^2 + A\text{-}virtual3[+offset3]^2$

sum b: $A\text{-}virtual1[-offset1]^2 + A\text{-}virtual2[-offset2]^2 + A\text{-}virtual3[-offset3]^2$

sum c: $A\text{-}virtual1^2 + A\text{-}virtual2^2 + A\text{-}virtual3^2$

Is sum "c" Max? — Yes → E

No ↓

Is sum "a" Max? — Yes →

Adjust virtual1, 2, and 3 axes by +offset and zero boxcar buffers

No ↓

Adjust virtual1, 2, and 3 axes by ⁻offset and zero boxcar buffers

↓

E

**Fig. 16c**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1887309 A1
- WO 02098273 A2
- EP 2168478 A1
- US 7697972 B2